(19) Europäisches Patentamt European Patent Office Office européen des brevets

(11) **EP 4 644 410 A1**

(12) **EUROPEAN PATENT APPLICATION**
published in accordance with Art. 153(4) EPC

(43) Date of publication:
**05.11.2025 Bulletin 2025/45**

(21) Application number: **23913046.1**

(22) Date of filing: **29.12.2023**

(51) International Patent Classification (IPC):
*C07K 14/47* (2006.01)   *C12N 15/62* (2006.01)
*A61K 39/00* (2006.01)   *A61P 35/00* (2006.01)

(52) Cooperative Patent Classification (CPC):
**A61K 38/00; A61K 39/00; A61P 35/00;
A61P 37/00; C07K 14/47; C12N 15/62**

(86) International application number:
**PCT/KR2023/022048**

(87) International publication number:
**WO 2024/144372 (04.07.2024 Gazette 2024/27)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  **30.12.2022  KR 20220191156**

(71) Applicant: **LG CHEM, LTD.**
**Seoul 07336 (KR)**

(72) Inventors:
• **CHO, Seong Je**
**Daejeon 34122 (KR)**
• **KIM, Dae Hee**
**Daejeon 34122 (KR)**

• **SHIN, Do Woon**
**Daejeon 34122 (KR)**
• **KIM, Youngmok**
**Daejeon 34122 (KR)**
• **KIM, Na Youn**
**Daejeon 34122 (KR)**
• **KIM, Da Eun**
**Daejeon 34122 (KR)**

(74) Representative: **Hoffmann Eitle
Patent- und Rechtsanwälte PartmbB
Arabellastraße 30
81925 München (DE)**

Remarks:
The complete document including Reference
Table(s) and the Sequence Listing(s) can be
downloaded from the EPO website

(54) **RECOMBINANT FUSION PROTEIN HAVING MODIFIED CYSTEINE RESIDUE FOR ANTIGEN DELIVERY AND USES THEREOF**

(57)    The present invention relates to: a fusion protein comprising a peptide antigen and a human thioredoxin protein linked to the N-terminus, the C-terminus or both of the peptide antigen, wherein all cysteine residues in the amino acid sequence of the human thioredoxin protein are substituted with non-cysteine residues; a nucleic acid molecule encoding the fusion protein; an expression vector comprising the nucleic acid molecule; a cell transformed with the expression vector; and a composition comprising the fusion protein, the nucleic acid molecule, the expression vector, or the cell.

【FIG. 21】

EP 4 644 410 A1

**Description**

[TECHNICAL FIELD]

Related are a fusion protein, comprising a peptide antigen, and a human thioredoxin protein linked to the N-terminus, C-terminus or both of the peptide antigen, wherein all cysteine residues in the amino acid sequence of the human thioredoxin protein are substituted with non-cysteine residues; a nucleic acid molecule encoding the fusion protein; an expression vector comprising the nucleic acid molecule; a cell transformed with the expression vector; and a composition comprising the fusion protein, nucleic acid molecule, expression vector, or cell.

[BACKGROUND ART]

Technologies to deliver antigens in various forms to induce T cell immunity have been developed, but there are advantages and disadvantages depending on the form. When antigens are delivered in a peptide form, there are disadvantages of difficult synthesis and purification depending on various antigen sequences and low exposure due to solubility and short half-life. When antigens are delivered in an mRNA form, there is an advantage of consistency in preparation, but formulation is necessary due to instability in the body, and there is a disadvantage that delivery of a certain amount of antigen (in vivo constant expression of various sequences) is uncertain. When antigens are delivered in a DNA form, in vivo stability is better than mRNA, but there are disadvantages that they should be delivered to the nucleus and have low antigen expression efficiency and DNA may be inserted into the genome. Viral vectors have advantages of in vivo delivery and high immunogenicity, but have disadvantages of decreased efficacy due to anti-drug antibodies in additional vaccination due to generation of strong antibodies against the viral surface.
Therefore, there is a need to develop a vaccine that can deliver antigens into the body more effectively and induce a stronger immune response.

[DISCLOSURE]

[TECHNICAL PROBLEM]

The present invention provides an antigen delivery system which enhances expression, physical properties, stability and/or immunogenicity of an antigen, and improves delivery of the antigen into immune response cells. In addition, the present invention provides an antigen delivery system which can prepare, express, purify and deliver sequences of various antigens, and can induce a stronger immune response in the body.
One embodiment provides a fusion protein, comprising a peptide antigen, and a human thioredoxin protein linked to the N-terminus, C-terminus or both of the peptide antigen, wherein at least 3 cysteine residues in the amino acid sequence of the human thioredoxin protein are substituted with non-cysteine residues.
Another embodiment, provides a nucleic acid molecule encoding the fusion protein.
Other embodiment, provides an expression vector comprising the nucleic acid molecule.
Other embodiment, provides a cell transformed with the expression vector.
Other embodiment, provides a method for preparing a fusion protein, comprising expressing the expression vector in a cell.
Other embodiment, provides a vaccine composition or immunogenic composition comprising the aforementioned fusion protein, nucleic acid molecule, expression vector, and/or cell.
Other embodiment, provides a method for causing, inducing, and/or promoting an immune response against an antigen, comprising administering an effective dose of the fusion protein, vaccine composition and/or immunogenic composition into a patient in need of stimulation of an immune response.
Other embodiment, provides a use of the fusion protein, vaccine composition and/or immunogenic composition for use in causing, inducing, and/or promoting an immune response against an antigen.
Other embodiment, provides a use of the fusion protein for use in the manufacture of the vaccine composition and/or immunogenic composition.
Other embodiment, provides a composition for use in preventing or treating disease comprising the fusion protein, nucleic acid molecule, expression vector, or cell.
Other embodiment, provides a method for preventing or treating disease, comprising administering the fusion protein and/or a composition comprising the fusion protein into a patient in need of prevention or treatment of disease.
Other embodiment, provides a use of the fusion protein and/or a composition comprising the fusion protein for use in prevention or treatment.
Other embodiment, provides a use of the fusion protein for use in the manufacture of a composition for use in preventing or treating disease.

Other embodiment, provides a composition for use in enhancing purification efficiency of a peptide antigen, comprising the aforementioned fusion protein, nucleic acid molecule, expression vector, or cell.

Other embodiment, provides a use of the fusion protein and/or a composition comprising the fusion protein, for enhancing purification efficiency of a peptide antigen.

Other embodiment, provides a method for preparation of a fusion protein, wherein at least 3 cysteine residues in the amino acid sequence of the human thioredoxin protein are substituted with non-cysteine residues, comprising linking the human thioredoxin protein to the N-terminus, C-terminus or both of the peptide antigen.

Other embodiment, provides a method for enhancing immunogenicity of a fusion protein, wherein at least 3 cysteine residues in the amino acid sequence of the human thioredoxin protein are substituted with non-cysteine residues, comprising preparing a fusion protein by linking the human thioredoxin protein to the N-terminus, C-terminus or both of the peptide antigen.

Other embodiment, provides a method for enhancing efficacy of preventing or treating diseases of a fusion protein, comprising preparing a fusion protein by linking the human thioredoxin protein to the N-terminus, C-terminus, or both of the peptide antigen, wherein at least 3 cysteine residues in the amino acid sequence of the human thioredoxin protein are substituted with non-cysteine residues.

Other embodiment, provides a method for enhancing purification efficiency of a fusion protein, comprising preparing a fusion protein by linking the human thioredoxin protein to the N-terminus, C-terminus, or both of the peptide antigen, wherein at least 3 cysteine residues in the amino acid sequence of the human thioredoxin protein are substituted with non-cysteine residues.

Other embodiment, provides a thioredoxin variant polypeptide consisting of the amino acid sequence of SEQ ID NO: 7 or SEQ ID NO: 9.

[TECHNICAL SOLUTION]

According to one aspect of the present invention, provided is a fusion protein, comprising a peptide antigen, and a human thioredoxin protein linked to the N-terminus, C-terminus or both of the peptide antigen, wherein at least 3 cysteine residues in the amino acid sequence of the human thioredoxin protein are substituted with non-cysteine residues.

As one embodiment, in the human thioredoxin protein, cysteine residues at the 62nd, 69th and 73rd amino acid positions in the amino acid sequence may be substituted with non-cysteine residues.

As one embodiment, in the human thioredoxin protein, cysteine residues at the 32nd, 35th, 62nd, 69th and 73rd amino acid positions in the amino acid sequence may be substituted with non-cysteine residues.

As one embodiment, the fusion protein may further comprise a carrier protein other than human thioredoxin at the N-terminus or C-terminus or both.

As one embodiment, in the fusion protein, an affinity tag may be linked to the N-terminus, C-terminus, or both.

As one embodiment, a linker may be present between the peptide antigen and human thioredoxin protein.

As one embodiment, the peptide antigen may comprise a T cell epitope.

As one embodiment, the peptide antigen may comprise a T cell epitope, derived from a tumor antigen, an antigen of an infection source, an autoantigen, or an allergy causing antigen.

As one embodiment, the carrier protein may be a protein which improves recombinant expression of the peptide antigen, or enhances the purification efficiency of the peptide antigen.

As one embodiment, the tumor antigen may include a tumor-associated antigen (TAA), a tumor-specific antigen (TSA) or a tumor-derived neoantigen.

As one embodiment, the tumor-derived neoantigen may comprise a mutation specifically expressed in a cancer cell.

As one embodiment, the tumor-associated antigen (TAA) may be CT (Cancer-testis) antigen, EGFR, M12, M20, M21, M30, M44, Ova, Melan-A, PSMA (Prostate Specific Membrane Antigen), Survivin, MAGE-A, ADAbp (adenosine deaminase-binding protein), cyclophilin b, gp100, CRC (Colorectal associated antigen)-C017-1 A/GA733, CEA (carcinoembryonic antigen), CAP-1, CAP-2, etv6, AML1, PSA (Prostate Specific Antigen), PSA-1, PSA-2, PSA-3, MAGE (melanoma antigen E), GAGE (G antigen), BAGE (B melanoma antigen), RAGE (renal tumor antigen), LAGE (L antigen), NAG, GnT-V, MUM-1, CDK4, p53, tyrosinase, Muc1 (Mucin1), HER2/neu, p21ras, RCAS1, $\alpha$-fetoprotein, E-cadherin, $\alpha$-catenin, $\beta$-catenin, $\gamma$-catenin, p120ctn, PRAME, NY-ESO-1, TRP2, Mammaglobin-A, metallopanstimulin-1 (MPS-1), cytochrome P450 isoform 1B1, 90K/Mac-2 binding protein, Ep-CAM (MK-1), HSP-70, hTERT (TRT), LEA, TAGE-1, 5T4, gp70, SCP-1, c-myc, cyclin B1, MDM2, p62, Koc, IMP1, TA90, OA1, CT-7, HOM-MEL-40/SSX-2, SSX-1, SSX-4, HOM-TES-14/SCP-1, HOM-TES-85, HDAC5, MBD2, TRIP4, NY-CO-45, KNSL6, HIP1R, Seb4D, KIAA1416, IMP1, 90K/Mac-2 binding protein, MDM2, or LMNA.

As one embodiment, the antigen of the infection source may be an antigen derived from a virus, bacterium, parasite, or fungus.

As one embodiment, the carrier protein may be one or more selected from NDPK (nucleoside diphosphate kinase B), CSTA (Cystatin-A), Trx (Thioredoxin), RPL7Am (50S ribosomal protein L7Ae), Samp2a (Small archaeal modifier protein 2), TE (Tenascin), TM1112 (*Thermotoga maritima* Cupin_3 domain-containing protein), TrxA (Thioredoxin 1), TTrx (*Thermosipho africanus* Thioredoxin), PSBD (peripheral subunit-binding domain), a fragment thereof, a variant thereof, a fragment of the variant thereof and a variant of the fragment thereof.

As one embodiment, the fragment of PSBD (peripheral subunit-binding domain) may comprise the amino acid sequence of SEQ ID NO: 13. The fragment of PSBD (peripheral subunit-binding domain) comprising the amino acid sequence of SEQ ID NO: 13 may be a variant of the fragment of PSBD.

As one embodiment, the affinity tag may be His or streptavidin.

As one embodiment, the linker may be (GS)n, (G₂S)n, (G₃S)n, (G₄S)n, Gn, LE, SSGG or GGGGSGGGGG (herein, G is Gly, S is Ser, L is Leu, E is Glu, n is an integer of at least 1).

As one embodiment, the fusion protein may have a size of 30kDa.

According to another aspect of the present invention, a nucleic acid molecule encoding the fusion protein is provided.

According to other aspect of the present invention, an expression vector comprising the nucleic acid molecule is provided.

According to other aspect of the present invention, a cell transformed with the expression vector is provided.

According to other aspect of the present invention, a vaccine composition comprising the aforementioned fusion protein, nucleic acid molecule, expression vector, or cell is provided.

According to other aspect of the present invention, an immunogenic composition comprising the aforementioned fusion protein, nucleic acid molecule, expression vector, or cell is provided.

As one embodiment, the compositions may further comprise an adjuvant.

According to other aspect of the present invention, provided is a method for causing, inducing and/or promoting an immune response against an antigen, comprising administering an effective dose of the fusion protein, vaccine composition and/or immunogenic composition into a patient.

According to other aspect of the present invention, provided is a method for causing, inducing, and/or promoting an immune response against an antigen, comprising administering an effective dose of the fusion protein, vaccine composition and/or immunogenic composition into a patient.

According to other aspect of the present invention, provided is a use of the fusion protein, vaccine composition, and/or immunogenic composition, for causing, inducing, and/or promoting an immune response against an antigen.

According to other aspect of the present invention, provided is a use of the fusion protein, for use in the manufacture of a vaccine composition and/or an immunogenic composition.

According to other aspect of the present invention, provided is a method for preventing or treating disease, comprising administering the fusion protein and/or a composition comprising the fusion protein into a patient in need of preventing or treating disease.

According to other aspect of the present invention, provided is a use of the fusion protein and/or a composition comprising the fusion protein for use in preventing or treating disease.

According to other aspect of the present invention, provided is a use of the fusion protein for use in the manufacture of a composition for preventing or treating disease.

According to other aspect of the present invention, provided is a composition for enhancing purification efficiency of a peptide antigen, comprising the aforementioned fusion protein, nucleic acid molecule, expression vector, or cell.

According to other aspect of the present invention, provided is a use of the fusion protein and/or a composition comprising the fusion protein for enhancing purification efficiency of a peptide antigen.

According to other aspect of the present invention, provided is a use of the fusion protein for use in the manufacture of a composition for enhancing purification efficiency of a peptide antigen.

Other embodiment, provides a method for preparing a fusion protein with enhanced immunogenicity compared to a fusion protein linked with a wild type human thioredoxin protein, comprising linking the human thioredoxin protein to the N-terminus, C-terminus, or both of the peptide antigen, wherein at least 3 cysteine residues of the amino acid sequence of the human thioredoxin protein are substituted with non-cysteine residues.

Other embodiment, provides a method for preparation of a fusion protein, wherein at least 3 cysteine residues of the amino acid sequence of the human thioredoxin protein are substituted with non-cysteine residues, comprising linking the human thioredoxin protein to the N-terminus, C-terminus, or both of the peptide antigen.

Other embodiment, provides a method for enhancing immunogenicity of a fusion protein, comprising preparing a fusion protein by linking the human thioredoxin protein at the N-terminus, C-terminus or both of the peptide antigen, wherein at least 3 cysteine residues of the amino acid sequence of the human thioredoxin protein are substituted with non-cysteine residues.

Other embodiment, provides a method for enhancing efficacy of treating or preventing disease of a fusion protein, comprising preparing a fusion protein by linking the human thioredoxin protein at the N-terminus, C-terminus or both of the peptide antigen, wherein at least 3 cysteine residues of the amino acid sequence of the human thioredoxin protein are substituted with non-cysteine residues.

Other embodiment, provides a method for enhancing purification efficiency of a fusion protein, comprising preparing a fusion protein by linking the human thioredoxin protein at the N-terminus, C-terminus or both of the peptide antigen, wherein at least 3 cysteine residues of the amino acid sequence of the human thioredoxin protein are substituted with non-cysteine residues.

According to other aspect of the present invention, a thioredoxin variant polypeptide consisting of the amino acid sequence of SEQ ID NO: 7 or SEQ ID NO: 9 is provided.

Hereinafter, the present invention will be described in more detail.

In the present description (including claims), when the terms "comprise (comprises)", "comprised" or "comprising" are used, they are interpreted as specifying presence of described features, integers, steps or components, but they should be interpreted as not excluding presence of other features, integers, steps, components more than that, or groups thereof.

In the present description, the descriptions of documents, laws, materials, devices, and articles and the like, are included only for the purpose of providing context for the present invention. All or any of them does not form a part of the prior art, or suggest or represent common general knowledge in the field to which the present invention pertains prior to the priority date of each claim of the present application.

One embodiment of the present application relates to a fusion protein, comprising a peptide antigen and a human thioredoxin (Trx) protein linked to the N-terminus, C-terminus or both of the peptide antigen, wherein at least 3 cysteine residues of the amino acid sequence of the human thioredoxin protein are substituted with non-cysteine residues.

In the present description, that protein (can be interchangeably used with "polypeptide") or polynucleotide (can be interchangeably used with "gene") "comprises a specific amino acid sequence or nucleic acid sequence" or "consists of (consisting of) or is represented by a specific nucleic acid sequence or amino acid sequence" may mean that the polypeptide or polynucleotide essentially comprises the specific nucleic acid sequence or amino acid sequence, and it may be interpreted as comprising "substantially equivalent sequences" in which a mutation (deletion, substitution, modification, and/or addition) is applied to the specific amino acid sequence or nucleic acid sequence.

In one embodiment, that polypeptide or polynucleotide "comprises a specific amino acid sequence or nucleic acid sequence" or "consists of or is represented by a specific amino acid sequence or nucleic acid sequence" may mean that the polypeptide or polynucleotide (i) essentially comprises the specific amino acid sequence or nucleic acid sequence, or (ii) consists of an amino acid sequence or nucleic acid sequence having identity of 70% or more, 75% or more, 80% or more, 85% or more, 90% or more, 91% or more, 92% or more, 93% or more, 94% or more, 95% or more, 96% or more, 97% or more, 98% or more , 99% or more, 99.5% or more, or 99.9% or more to the specific amino acid sequence or nucleic acid sequence, or essentially comprises the same, and maintains the original functions and/or desired functions. In the present description, that the polypeptide, peptide antigen, human thioredoxin protein, further comprised carrier protein, and fusion protein "comprises a specific amino acid sequence or is represented by or consists of a specific amino acid sequence" may mean both the case of essentially comprising the amino acid sequence, and the case of introducing an insignificant mutation (for example, substitution, deletion, and/or addition of an amino acid residue) which does not affect the original activity and/or desired activity (for example, immunogenicity, enhancement of purification efficiency, activity of preventing or treating disease, etc.) in the amino acid sequence.

In the present description, the term "identity" refers to the degree of being consistent to the given nucleic acid sequence or amino acid sequence, and may be expressed as a percentage (%). In case of identity to a nucleic acid sequence, for example, it may be determined using algorithm BLAST by the document (See: Karlin and Altschul, Pro. Natl. Acad. Sci. USA, 90, 5873, 1993) or FASTA by Pearson (See: Methods Enzymol., 183, 63, 1990). Based on this algorithm BLAST, programs called BLASTN or BLASTX have been developed (See: http://www.ncbi.nlm.nih.gov).

The term, "antigen" refers to any molecule that induces an immune response in an object. As one example, the antigen refers to any molecule containing an epitope which can be recognized by a T cell receptor and/or a B cell receptor, and can stimulate an immune response, in particular, a T cell response and/or a B cell response in an object.

The term, "epitope" refers to a region of an antigen that interacts with a T-cell receptor and/or B-cell receptor.

As one preferable embodiment, the peptide antigen of the present application may comprise a T cell epitope. For example, the peptide antigen of the present application may comprise MHC class I and/or II binding motifs. As a specific embodiment, the peptide antigen of the present application may comprise a CD4+ T cell epitope which is a peptide sequence that comprises the MHC class II binding motif and can be suggested on the surface of an antigen-presenting cell by the MHC class II molecule. The peptide antigen of the present application may also comprise a CD8+ T cell epitope which is a peptide sequence that comprises the MHC class I binding motif and can be suggested on the cell surface by the MHC class I molecule. The peptide antigen of the present application may also comprise both the CD4+ T cell epitope and CD8+ T cell epitope.

The term, "MHC (major histocompatibility complex)" or "major histocompatibility complex" is a protein that plays a role of providing an antigen fragment into an immunocyte so as to distinguish non-self molecules, and there are two types of MHC class I and MHC class II, and whereas MHC class I is present in all cells having a nucleus, MHC class II is found in antigen-presenting cells. MHC class I molecules interact with CD8+ cytotoxic T cells, and play an important role in occurring rejection reaction of organ transplant or destroying infected cells. MHC class II molecules play an important role in causing cell-mediated immunity by recognizing non-self antigens through interaction with CD4+ helper T cells.

In general, in an adaptive immune response, when an antigen enters the body, antigen-presenting cells ingest it and decompose it into short peptide fragments, and peptides can bind to MHC class I or MHC class II molecules and be transported to the cell surface. In this way, when the peptides of the antigen bind to MHC class I or MHC class II and are presented on the cell surface of the antigen-presenting cells, T cells recognize them by a T cell receptor (TCR) and are activated and initiate an immune response. In this respect, the antigen protein herein may correspond to an epitope of T cells.

In another preferable embodiment, the peptide antigen may be a peptide derived from a tumor antigen, for example, a tumor-associated antigen (TAA), a tumor-specific antigen (TSA), or a tumor-derived neoantigen; an antigen of an infection source, for example, an antigen derived from a virus, bacterium, parasite, or fungus; an autoantigen known or suspected to cause autoimmunity; or an allergy causing antigen (allergen) known or suspected to cause allergy, but not limited thereto.

For example, the peptide antigen may comprise a part of a tumor antigen, an antigen of an infection source, an autoantigen, or an allergy causing antigen, which is predicted in silico or known to bind to an MHC class I or MHC class II molecule. These antigens may be a CD8+ or CD4+ T cell epitope, but not limited thereto.

The peptide antigen may comprise a natural or non-natural amino acid sequence capable of inducing an immune response, for example, a T cell or B cell response, in an object, an amino acid having modification after translation, or a peptide mimetic. For example, the antigen protein (for example, T cell epitope) may be 5 to 100, or 5 to 50 amino acids. For example, the peptide antigen may be 7 to 35 amino acids, for example, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or 35 amino acids, but not limited thereto.

The term "tumor antigen" may be interchangeably used with "cancer antigen", and refers to an antigen expressing in tumor (cancer), causing an immune response. This immune response may accompany antibody generation, or activation of a specific immune-competent cell, or both of them.

The tumor antigen may be derived from an organism with tumor, entire killed or non-activated tumor cells, or lysates, and includes any antigen derived from tumor. The lysates refer to substances generated from processing disruption of a normal structure of a cell. In addition, the tumor antigen includes any protein or other substance having antigen characteristics, which is comprised in tumor cells and is expressed differently from normal cells.

The tumor antigen may comprise a tumor-associated antigen (TAA), a tumor-specific antigen (TSA) or a tumor-derived neoantigen.

The tumor-associated antigen (TAA) is an antigen which appears more frequently in cancer cells than normal cells or appears at a differentiation stage different from normal cells, and is a tumor shared antigen that is present in a trace amount also in normal cells. Therefore, there is a high possibility that an immune response using this may be nullified by auto-tolerance, which is an immunosuppressive mechanism to prevent damage of self cells, or, on the contrary, that organs may be attacked due to autoimmunity.

Examples of the tumor-associated antigen (TAA) include EGFR, Ova, Melan-A, PSMA (Prostate Specific Membrane Antigen), Survivin, MAGE-A, ADAbp (adenosine deaminase-binding protein), cyclophilin b, gp100, CRC (Colorectal associated antigen)-C017-1 A/GA733, CEA (carcinoembryonic antigen), CAP-1, CAP-2, etv6, AML1, PSA (Prostate Specific Antigen), PSA-1, PSA-2, PSA-3, MAGE (melanoma antigen E)[for example, MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A12, MAGE-Xp2(MAGE-B2), MAGE-Xp3(MAGE-B3), MAGE-Xp4(MAGE-B4), MAGE-C1, MAGE-C2, MAGE-C3, MAGE-C4, MAGE-C5, etc.], GAGE (G antigen) [for example, GAGE-1, GAGE-2, GAGE-3, GAGE-4, GAGE-5, GAGE-6, GAGE-7, GAGE-8, GAGE-9, etc.], BAGE (B melanoma antigen), RAGE (renal tumor antigen), LAGE (L antigen), NAG, GnT-V, MUM-1, CDK4, p53, tyrosinase, Muc1 (mucin1), HER2/neu, p21ras, RCAS1, $\alpha$-fetoprotein, E-cadherin, $\alpha$-catenin, $\beta$-catenin, $\gamma$-catenin, p120ctn, PRAME, NY-ESO-1, TRP2, Mammaglobin-A, metallopanstimulin-1 (MPS-1), cytochrome P450 isoform 1B1, 90K/Mac-2 binding protein, Ep-CAM (MK-1), HSP-70, hTERT (TRT), LEA, TAGE-1, 5T4, gp70, SCP-1, c-myc, cyclin B1, MDM2, p62, Koc, IMP1, TA90, OA1, CT-7, HOM-MEL-40/SSX-2, SSX-1, SSX-4, HOM-TES-14/SCP-1, HOM-TES-85, HDAC5, MBD2, TRIP4, NY-CO-45, KNSL6, HIP1R, Seb4D, KIAA1416, IMP1, 90K/Mac-2 binding protein, MDM2, or LMNA, but not limited thereto.

The tumor-specific antigen (TSA) refers to an antigen present only in cancer cells. In particular, when tumor is proliferated in cancer patients, cancer cell-specific gene mutation occurs, generating a new antigen epitope capable of stimulating T cells, and this is called a neoantigen. In other words, the neoantigen may comprise a cancer cell-specific gene mutation, and is selectively expressed only in cancer cells, unlike tumor shared antigens expressed in a trace amount also in normal cells, and thus, it is recognized as a non-self foreign epitope by the autoimmune system, and induces strong anti-cancer immune activity.

When peptides generated from mutated DNA are displayed on the MHC on the cell surface, a T cell receptor (TCR) recognizes it, but mutation does not occur in normal cells or tissues, so neoantigen-specific T cells are free from self-tolerance or autoimmune problems. Due to such advantages, the neoantigen is considered an ideal target for T cell-based cancer immunotherapy.

Causes of generating neoantigens include frame-shift (frame-shift deletion or insertion) in which translation of genetic codes is dislocated as one or more nucleotides consisting of DNA are added or deleted, point mutation occurring by replacing one nucleotide with another one, as well as missense mutation, splice-site mutation, read-through mutation, or gene-fusion mutation or the like, but are not limited thereto.

Neoantigens are predicted through specific cancer cell genome analysis of an individual cancer patient, and as one example, cancer cells are obtained from a patient's tumor, and DNA is extracted, and then base sequences are analyzed, and these are compared and analyzed with base sequences of a normal cell, and then among base sequences of various mutation-occurred portions, neoantigens that could stimulate T cells may be identified. For this, processing of big data such as next generation sequencing (NGS), whole-exome sequencing (WES), or RNA sequencing, a computer program for predicting MHC binding, or artificial intelligence (AI) for prediction of neoantigens, or the like, may be used, but not limited thereto. Since mutant forms are not shared between patients, neoantigens can be produced as a personalized cancer vaccine.

In one embodiment, the tumor-derived neoantigen may comprise the amino acid sequence selected from the group consisting of SEQ ID NO: 1 to SEQ ID NO: 5.

In one embodiment, the antigen of the infection source may be an antigen derived from a virus, bacterium, parasite, or fungus, but not limited thereto.

For example, the virus-derived antigen may be an antigen derived from poxvirus, variola virus, Ebola virus, Marburg virus, dengue virus, influenza virus, parainfluenza virus, respiratory syncytial virus, measle virus, human immunodeficiency virus, human papillomavirus, varicella-zoster virus, herpes simplex virus, cytomegalovirus, Epstein-Barr virus, JC virus, rhabdovirus, rotavirus, rhinovirus, adenovirus, papilloma virus, parvovirus, picornavirus, poliovirus, mumps causing virus, rabies causing virus, reovirus, rubella virus, togavirus, orthomyxovirus, retrovirus, hepadnavirus, coxsackievirus, equine encephalitis virus, Japanese encephalitis virus, yellow fever virus, Rift Valley fever virus, hepatitis A virus, hepatitis B virus, hepatitis C virus, hepatitis D virus or hepatitis E virus, but not limited thereto.

The bacterium-derived antigen may be an antigen derived from Borrelia species, Bacillus anthracis, burgdorferi, Bordetella pertussis, Camphylobacter jejuni, Chlamydia species, Chlamydial psittaci, Chlamydial trachomatis, Clostridium species, Clostridium tetani, Clostridium botulinum, Clostridium perfringens, Corynebacterium diphtheriae, Coxiella species, Enterococcus species, Erlichia species, Escherichia coli, Francisella tularensis, Haemophilus species, Haemophilus influenzae, Haemophilus parainfluenzae, Lactobacillus species, Legionella species, Legionella pneumophila, Leptospirosis interrogans, Listeria species, Listeria monocytogenes, Mycobacterium species, Mycobacterium tuberculosis, Mycobacterium leprae, Mycoplasma species, Mycoplasmapneumoniae, Neisseria species, Neisseria meningitidis, Neisseria gonorrhoeae, Pneumococcus species, Pseudomonas species, Pseudomonas aeruginosa, Salmonella species, Salmonella typhi, Salmonella enterica, Rickettsia species, Rickettsia ricketsii, Rickettsia typhi, Shigella species, Staphylococcus species, Staphylococcus aureus, Streptococcus species, Streptococccus pnuemoniae, Streptococcus pyrogenes, Streptococcus mutans, Treponema species, Treponema pallidum, Vibrio species, Vibrio cholerae, or Yersinia pestis, but not limited thereto.

The fungus-derived antigen may be a fungus-derived antigen selected from Candida species, Cryptococcus species, Coccidioides species, Histoplasma species and Aspergillus species, but not limited thereto.

The parasite-derived antigen may be an antigen derived from Plasmodium, Trypanosome, Schistosome or Leishmania, but not limited thereto.

Thioredoxin (Trx) is a redox protein with a low molecular weight of about 11-12 kDa found in both prokaryotic cells and eukaryotic cells, and is reduced to Trx-$(SH)_2$ reversibly by NADPH and thioredoxin reductase. While human thioredoxin (hTrx) consists of 105 amino acids (SEQ ID NO: 10), and has 5 cysteine (Cys) residues at the 32nd, 35th, 62nd, 69th and 73rd amino acid positions, E. coli thioredoxin (TrxA) consists of 109 amino acids (SEQ ID NO: 11), and has 2 cysteine (Cys) at the 32nd and 35th amino acid positions. Human thioredoxin has the amino acid identity of 27% to E. coli thioredoxin. Thioredoxin is known to act as an electron donor for ribonucleotide reductase, methionine sulfoxide reductase, 3-phosphoadenosine 5'phosphosulfate reductase and the like, to facilitate refolding of protein comprising -S-S- bonds, and to function as an antioxidant and the like.

The human thioredoxin protein may be linked to the N-terminus, C-terminus or both of the peptide antigen, and it may be linked to each component such as the peptide antigen, the further comprised carrier protein or affinity tag of the present invention or the like through a linker.

Herein, the human thioredoxin protein may function as a carrier protein that improves recombinant expression of the peptide antigen, enhances purification efficiency, improves physical properties, stabilizes, increases immunogenicity, and/or improves delivery into immune response cells by being linked to the N-terminus, C-terminus or both of the peptide antigen. In particular, herein, it is characterized by a variant wherein at least 3 cysteine residues in the amino acid sequence of the human thioredoxin protein are substituted with non-cysteine residues.

Specifically, the human thioredoxin protein may be a variant wherein cysteine residues at the 32nd, 35th, and 62nd; 32nd, 35th and 69th; 32nd, 35th and 73rd; 32nd, 62nd and 69th; 32nd, 62nd and 73rd; 32nd, 69th and 73rd; 35th, 62nd and 69th; 35th, 62nd and 73rd; 35th, 69th and 73rd; 62nd, 69th and 73rd; 32nd, 35th, 62nd and 69th; 32nd, 35th, 62nd and 73rd; 32nd, 35th, 69th and 73rd; 32nd, 62nd, 69th and 73rd; 35th, 62nd, 69th and 73rd; or 32nd, 35th, 62nd, 69th, and 73rd amino acid positions in the amino acid sequence of the human thioredoxin protein are substituted with non-cysteine residues, but not limited thereto. As a more preferable embodiment, it may be a variant wherein 3 cysteine (Cys) residues at the 62nd, 69th and 73rd amino acid positions or all of 5 cysteine (Cys) residues at the 32nd, 35th, 62nd, 69th, and 73rd amino acid positions in the amino acid sequence of the human thioredoxin protein are substituted with non-cysteine residues, but not limited thereto.

The human thioredoxin protein variant wherein at least 3 cysteine residues in the amino acid sequence of the human thioredoxin protein are substituted with non-cysteine residues reduces dimerization by disulfide bonds while maintaining functions as a carrier protein. Therefore, a fusion protein prepared by linking the human thioredoxin protein variant to a peptide antigen may reduce generation of multimers during purification to enhance purification efficiency, increase structural stability, ultimately be efficiently delivered to immunocytes, and/or enhance immunogenicity.

Cysteine residues may be substituted with another amino acid residues which are not involved in disulfide bond formation. Therefore, they may be substituted with other amino acids other than cysteine (i.e., non-cysteine residues). More preferably, cysteine residues may be substituted with alternative residues that preserves the overall structure such as serine or alanine, but not limited thereto. For example, the human thioredoxin protein variant may consist of the amino acid sequence of SEQ ID NO: 7 or SEQ ID NO: 9, but not limited thereto.

Other embodiment of the fusion protein of the present application, may further comprise or not comprise a carrier protein other than human thioredoxin at the N-terminus or C-terminus or both of the fusion protein.

The term, "carrier protein" refers to a protein which is linked to a peptide antigen to improve recombinant expression of the peptide antigen (for example, high expression rate, consistent expression rate, etc.), enhance purification efficiency, improve physical properties, stabilize, enhance immunogenicity, and/or improve delivery into immune response cells.

One or more of the carrier protein further comprised may be linked to the N-terminus, C-terminus or both of the fusion protein, respectively. For example, 1, 2, or 3, or more of the carrier protein further comprised may be linked to the N-terminus, C-terminus, or both, respectively.

As one embodiment, when the human thioredoxin protein is linked to the N-terminus of the peptide antigen, at least one carrier protein further comprised may be linked to at least one position selected from the group consisting of the followings:

N-terminus of the human thioredoxin protein;
Between the C-terminus of the human thioredoxin protein and the N-terminus of the peptide antigen; and
C-terminus of the peptide antigen.

As one embodiment, when the human thioredoxin protein is linked to the C-terminus of the peptide antigen, at least one carrier protein further comprised may be linked to at least one position selected from the group consisting of the followings:

N-terminus of the peptide antigen;
Between the C-terminus of the peptide antigen and the N-terminus of the human thioredoxin protein; and
C-terminus of the human thioredoxin protein.

As one embodiment, when the human thioredoxin protein is linked to the N-terminus and C-terminus of the peptide antigen, at least one carrier protein further comprised may be linked to at least one position selected from the group consisting of the followings:

N-terminus of the first human thioredoxin protein;
Between the C-terminus of the first human thioredoxin protein and the N-terminus of the second human thioredoxin protein; and
C-terminus of the peptide antigen,
wherein the first human thioredoxin protein is a human thioredoxin protein linked to the N-terminus or N-terminal direction of the peptide antigen, and the second human thioredoxin protein is a human thioredoxin protein linked to the C-terminus or C-terminal direction of the peptide antigen.

Non-restrictive examples of the carrier protein further comprised in the fusion protein of the present invention may be NDPK (nucleoside diphosphate kinase B), CSTA(Cystatin-A) [for example, human CSTA (hCSTA) or mouse CSTA (mCSTA), etc.], Trx (Thioredoxin) [for example, Trx of mammals or bacteria, etc.], RPL7Am (50S ribosomal protein L7Ae), Samp2a (Small archaeal modifier protein 2), TE (Tenascin)[for example, TE1, TE2, TE3.1, TE3.2, TE4, etc.], TM1112 (*Thermotoga maritima* Cupin_3 domain-containing protein), TrxA (Thioredoxin 1), TTrx (*Thermosipho africanus* Thioredoxin), or PSBD(peripheral subunit-binding domain), or be at least one of fragments or variants thereof, fragments of the variants or variants of the fragments, but not limited thereto. In addition, they may be codon-optimized for host cells, or comprise mutations for some sequences for the purpose of improving functions or structural stability, or be appropriately modified to comprise initiation methionine. For example, the modification may be introduced by deleting, substituting or adding some of the sequence, for a purpose of removing residues capable of being involved in generation of dimerization or redox reactions, inhibiting nucleic acid binding, inhibiting binding to Desampylase (UniProt: Q8U1Y4), reducing excessive binding affinity to human serum albumin, facilitating quantification, or inhibiting deamidation, or the like, but is not limited thereto.

As a preferable embodiment, for the carrier protein positioned at the N-terminus, the carrier protein may be selected in aspect of improving expression of an antigen, considering problems of difficult recombinant expression of the antigen depending on the sequence of the tumor antigen. For example, the carrier protein at the N-terminus may be selected for the purpose of expressing an antigen above a certain level, and/or consistently expressing it and/or stabilizing it in a host expression system, but not limited thereto.

In addition, as a preferable embodiment, for the carrier protein positioned at the C-terminus, the carrier protein may be selected in aspect of enhancing purification efficiency of an antigen, considering problems of difficult purification (or removal of impurities) of the antigen depending on the sequence of the tumor antigen. For example, the carrier protein at the C-terminus may be selected for the purpose of enhancing purification efficiency by stabilizing a fusion protein C-terminal structure in a common purification system, but not limited thereto.

Furthermore, the carrier protein may include pan HLA DR binding epitopes (PADRE), tetanus toxoids (Tetanus toxin epitopes, TT) or diphtheria toxoids or recombinantly produced, and genetically detoxified variants thereof, Staphylococcus exotoxins or toxoids, or Pseudomonas aeruginosa exotoxin A or derivatives thereof, or the like, but not limited thereto.

The carrier protein further comprised may be linked to each component such as the peptide antigen, human thioredoxin protein, other carrier protein further comprised or an affinity tag of the present invention, or the like, through a linker.

Other embodiment of the fusion protein of the present application, may further comprise or not comprise an affinity tag at the N-terminus, C-terminus or both of the fusion protein.

The term, "affinity tag" refers to a substance that provides a site for attachment of the fusion protein to a specific substrate, when the fusion protein is purified in vitro, that is, experimentally. The affinity tag is a part of the fusion protein, and it should not cause immunogenicity or affect the activity of the fusion protein.

The fusion protein of the present application can be purified regardless of characteristics depending on the antigen sequence by comprising an affinity tag, and superhigh speed protein production may be possible by making the fusion protein easy to be purified through affinity purification.

The affinity tag may be poly histidine (His), poly phenylalanine, poly alanine, streptavidin, Maltose Binding Protein (MBP), Intein, thioredoxin (Trx), protein A, NusA (N utilization substance A), beta-galactosidase (β-galactosidase), or Glutathione-S-transferase (GST), but not limited thereto. For example, in case of the poly histidine tag, a peptide of the sequence with consecutive histidine of 5 to 8 is used (for example, His-His-His-His-His-His), and the histidine tag has affinity to divalent metal ions, so the fusion protein can be purified by affinity chromatography using Nickel immobilized bead, Cobalt immobilized bead, and the like.

In one embodiment, the affinity tag may be 6-His.

Affinity tags may be cleaved or not cleaved under in vitro or in vivo conditions. Even if the affinity tags are not cleaved, they are safe in vivo, do not cause structural changes in the fusion protein, and do not substantially affect exhibiting its function of the fusion protein in vivo.

As a preferable embodiment, the affinity tag may be linked to the C-terminus to enable antigens to be purified regardless of antigen sequence characteristics, but not limited thereto.

The affinity tag may be liked to each component such as the peptide antigen, human thioredoxin protein or carrier protein further comprised of the present invention, or the like through a linker.

In other embodiment of the fusion protein of the present application, a linker may be present between each component such as the peptide antigen, human thioredoxin protein, carrier protein, affinity tag and the like.

The term, "linker" refers to a molecule or atom group which links or couples or binds at least two components together. Herein, each component of the fusion protein, for example, a peptide antigen and a thioredoxin protein, a peptide antigen and a carrier protein, a thioredoxin protein and a carrier protein, or a carrier protein and an affinity tag may be linked or combined together through any appropriate means, respectively. The linker may have additional functions such as increasing or reducing water solubility, or increasing the distance between two components to be linked to provide flexibility or increasing stability, but it is preferable that it does not cause immunogenicity or affect the activity of the fusion protein.

The linker may be a peptide linker, and may be 1 to 10, or 2 to 10 amino acids, for example, 1, 2, 3, 4, 5, 6, 7, 8, 9, 10 or more (for example, 20 or less) amino acids in length, for example, 11, 12, 13, 14, 15, 16, 17, 18, 19, 20 amino acids in length, but not limited thereto. As one example, the peptide linker may consist of neutral amino acids (more specifically, Gly, Ser, Ala, Thr or combination of these 4 amino acids). For example, it may (GS)n, (G$_2$S)n, (G$_3$S)n, (G$_4$S)n, Gn, LE, SSGG or GGGGSGGGGG (herein, G is Gly, S is Ser, L is Leu, E is Glu, n is an integer of 1 to 10, for example, 1, 2, 3, 4, 5, 6, 7, 8, 9 or 10, but not limited thereto), and for example, it may be GS, GGGGS, LE, SSGG, GG, GGGGG or GGGGSGGGGG, but not limited thereto.

In one embodiment, the linker may comprise a degradable peptide sequence cleavable by intracellular enzyme, for example, protease, and the cleavage of the linker may cause exposure of any component linked to the linker, for example, an affinity tag, a thioredoxin protein or a carrier protein.

In one embodiment, the linker may comprise at least one amino acid sequence selected from the group consisting of SEQ ID NO: 51 to SEQ ID NO: 52.

The term, "linked" means that components are directly or indirectly linked together. Each component may be linked covalently or non-covalently. In addition, "linked" may mean maintaining chemical or physical bonds of each of the components, after contacting and immunizing cells, for example, antigen-presenting cells or immunocytes. For example, until the antigen-presenting cells and immunocytes are in contact, components may be associated so that they cannot freely disperse from each other. For example, two components may be linked covalently to each other, so that these two components cannot disperse or diffuse separately.

In one embodiment, the size of the fusion protein of the present application is not particularly limited, but a small size may be advantageous, for example, for the purpose of facilitating the removal of impurities during the purification process. In this aspect, the overall size of the fusion protein may be in a size of 500 kDa or less, 400 kDa or less, 300 kDa or less, 200 kDa or less, 100 kDa or less, 90 kDa or less, 80 kDa or less, 70 kDa or less, 60 kDa or less, 50 kDa or less, for example, 10 kDa to 100 kDa, 10 kDa to 90 kDa, 10 kDa to 80 kDa, 10 kDa to 70 kDa, 10 kDa to 60 kDa, 10 kDa to 50 kDa, 10 kDa to 40 kDa, 10 kDa to 30 kDa, but not limited thereto.

In one embodiment, the fusion protein of the present application may comprise an amino acid sequence selected from the group consisting of SEQ ID NO: 14 to SEQ ID NO: 50.

Other embodiment of the present application, relates to a thioredoxin variant polypeptide consisting of the amino acid sequence of SEQ ID NO: 7. This corresponds to the variant polypeptide according to one example of the present application wherein 3 cysteine (Cys) residues are substituted with serine at the 62nd, 69th and 73rd amino acid positions of the human thioredoxin protein. Other embodiment of the present application, relates to a thioredoxin variant polypeptide consisting of the amino acid sequence of SEQ ID NO: 9. This corresponds to the variant polypeptide according to one example of the present application wherein 5 cysteine (Cys) residues are substituted with serine at the 32nd, 35th, 62nd, 69th and 73rd amino acid positions of the human thioredoxin protein.

[Table 1]

| | |
|---|---|
| hTrx.v1 | MVKQIESKTAFQEALDAAGDKLVVVDFSATWCGPCKMIKPF FHSLSEKYSNVIFLEVDVDD**S**QDVASE**S**EVK**S**MPTFQFFKK GQKVGEFSGANKEKLEATINELV (SEQ ID NO: 7) |
| hTrx.v2 | MVKQIESKTAFQEALDAAGDKLVVVDFSATWCGPCKMIKPF FHSLSEKYSNVIFLEVDVDDCQDVASECEVK**S**MPTFQFFKK GQKVGEFSGANKEKLEATINELV- Mutant (C73S) |

(continued)

| hTrx.v3 | MVKQIESKTAFQEALDAAGDKLVVVDFSATW**S**GP**S**KMIKPF<br><br>FHSLSEKYSNVIFLEVDVDD**S**QDVASE**S**EVK**S**MPTFQFFKK GQKVGEFSGANKEKLEATINELV<br><br>(SEQ ID NO: 9) |
| --- | --- |
| hTrx (wild type) | MVKQIESKTAFQEALDAAGDKLVVVDFSATWCGPCKMIKPF<br><br>FHSLSEKYSNVIFLEVDVDDCQDVASECEVKCMPTFQFFKK<br><br>GQKVGEFSGANKEKLEATINELV<br><br>(SEQ ID NO: 10) |

Other embodiment of the present application, relates to a nucleic acid molecule encoding the fusion protein. The term, "nucleic acid molecule", "nucleic acid", or "nucleic acid sequence" refers to a polymer of deoxyribonucleotides or ribonucleotides present in a single-stranded or double-stranded form. The nucleic acid molecule encompasses RNA genome sequences, cDNA and RNA sequences transcribed therefrom, and unless otherwise mentioned particularly, it includes analogues of a natural nucleic acid.

The nucleic acid molecule includes not only nucleic acid sequences encoding the amino acid sequence of the fusion protein, but also complementary sequences to the sequence. The complementary sequences include not only completely complementary sequences, but also substantially complementary sequences, and this means a sequence that can be hybridized with for example, a nucleic acid sequence encoding the amino acid sequence of the fusion protein, under stringent conditions known in the art.

The nucleic acid molecule may be an isolated nucleic acid molecule.

The fusion protein of the present application is not limited thereto, but preferably, it may be obtained by expressing and purifying it by a recombinant method. Therefore, the present invention also provides an expression vector comprising a nucleic acid molecule encoding a fusion protein, and a cell transformed thereby, for expression and purification of the fusion protein.

Other embodiment of the present application relates to an expression vector comprising the nucleic acid molecule.

The term, "expression vector" refers to a nucleic acid structure operably linked to express a genetic insert encoding a target protein. As one embodiment, the expression vector may be linear or circular, or single-stranded or double-stranded DNA, cDNA, RNA and the like encoding at least two target proteins. The expression vector may form a part of a vector which can be used for transformation, transduction or transfection, but not limited thereto, and it may be transcribed and/or translated in itself in vitro.

The term, "operably linked" means that binding between nucleic acid sequences is functionally related. For example, a coding sequence (e.g., a sequence encoding a target protein) may be operably linked to appropriate regulatory elements to allow replication, transcription and/or translation thereof. For example, the coding sequence is operably linked to a promoter when the promoter can drive transcription of the coding sequence. The regulatory components need not be adjacent to the coding sequence as long as these function properly. For example, an intervening sequence that is not translated, but is transcribed, may be present between the promoter sequence and coding sequence, and the promoter sequence may still be considered "operably linked" to the coding sequence.

Each component in the expression vector should be operably linked to each other, and the linkage of these component sequences may be performed by ligation (linkage) at a convenient restriction enzyme site, and when such a site is not present, it may be performed using a synthetic oligonucleotide adaptor or linker according to a common method.

The expression vector may comprise transcription and translation expression regulatory sequences that enable the corresponding gene to be expressed in a host. The expression regulatory sequence may include a promoter for conducting transcription, any operator sequence for regulating such transcription, and/or sequences that regulate termination of transcription and translation. An initiation codon and a termination codon are generally considered to be a part of a nucleic acid sequence encoding a target protein, and should show activity in a subject when a genetic construct is administered, and should be in frame with a coding sequence.

For example, the promoter means a DNA sequence site to which transcription regulatory factors are combined, and for the purpose of the present invention, to increase the gene expression rate, a promoter capable of inducing strong and stable gene expression may be used.

The promoter may be constitutive or inducible. The promoter is not limited thereto, but early and late promoters of adenovirus, simian virus 40 (SV40) promoter, mouse mammary tumor virus (MMTV) promoters, long terminal repeat (LTR) promoters of HIV, Moloney virus promoter, cytomegalovirus (CMV) promoters, Epstein virus (EBV) promoters, Rous sarcoma virus (RSV) promoters, RNA polymerase $\pm$ promoters, T3 and T7 promoters, major operator and promoter regions of phage lambda and the like may be exemplified.

In addition, the expression vector may suitably comprise an adaptor or a linker, an enhancer, a selection marker (for example, antibiotic-resistant marker), a replicable unit, a poly A sequence, a tag for purification or other sequences of composition and induction known to regulate expression of genes of prokaryotic cells or eukaryotic cells or viruses thereof and various combinations thereof and the like.

For the expression vector, various forms of vectors such as plasmids, virus vectors, bacteriophage vectors, cosmid vectors, and the like may be used.

Other embodiment of the present application relates to a cell transformed with the expression vector.

In one embodiment, the transformed cell may be an isolated transformed cell.

In the present invention, the transformed cell may use any host cell known in the art as long as it is a host cell which can clone or express the expression vector stably and consecutively, and the prokaryotic cells include E. *coli,* for example, E. *coli* JM109, *E. coli* BL21, E. *coli* RR1, E. *coli* LE392, E. *coli B, E. coli* X 1776, *E. coli* W3110, Bacillus sp. strains such as Bacillus subtilis, and Bacillus thuringiensis, and Enterobacteriaceae strains such as Salmonella typhimurium, Serratia marcescens, and various Pseudomonas species, and the like, and when transformed into eukaryotic cells, as a host cell, yeasts *(Saccharomyces cerevisiae),* insect cells, plant cells and animal cells, for example, CHO cell lines (Chinese hamster ovary), W138, BHK, COS-7, 293, HepG2, 3T3, RIN and MDCK cell lines and the like may be used, but not limited thereto.

Introducing an expression vector into a cell may use a suitable standard technology known in the art, for example, electroporation, electroinjection, microinjection, calcium phosphate co-precipitation, calcium chloride/rubidium chloride method, retroviral infection, DEAE-dextran, cationic liposome method, polyethylene glycol-mediated uptake, gene gun and the like, but not limited thereto. Then, a circular structure may be introduced in a linear form by cleaving it with appropriate restriction enzyme.

The method for selecting the transformed cell may be performed easily according to a method widely known in the art, using a phenotype expressed by a selection marker. For example, when the selection marker is a specific antibiotic-resistant gene, a transformant may be easily selected by culturing the transformant in a medium containing the antibiotic.

Culturing of the transformed cell may be performed by various methods known in the art. For example, a protein secreted into cells or media may be obtained by conducting culturing by inoculating the transformed cell in a culture medium, and then adding IPTG into media when the cell density reaches a certain level to induce protein expression and culture it.

The protein secreted into cells or media may be obtained in a form purified according to various purification methods known in the art, but preferably, it may be purified by affinity chromatography using an affinity tag. For example, a desired protein may be easily obtained using a glutathione-combined resin column when the fusion protein is fused to GST, and IMAC (immobilized Metal Affinity Chromatography) when it is fused to His.

Other embodiment of the present application, provides a method for preparation of a fusion protein, comprising expressing the nucleic acid molecule or the expression vector in cells.

In one embodiment, a fusion protein prepared by the method for preparation may have improved purification efficiency, enhanced immunogenicity, and/or enhanced efficacy of preventing or treating disease, compared to a fusion protein in which a wild type human thioredoxin protein is linked.

A fusion protein may be prepared, by expressing a nucleic acid molecule encoding the fusion protein provided in the present description in an appropriate host cell described above.

In one embodiment, the method for preparation of a fusion protein may comprise culturing a cell comprising the nucleic acid molecule or the expression vector under conditions suitable for expression. The culturing may be performed under common culturing conditions as described above. In addition, the method for preparation may further comprise isolating and/or purifying a fusion protein from culture, after the culturing.

Other embodiment of the present application, relates to a composition for improving purification efficiency of a peptide antigen, comprising the aforementioned fusion protein, nucleic acid molecule, expression vector, or cell. The fusion protein of the present application can provide structural stability by reducing generation of multimers during purification of the fusion protein with the peptide antigen and improving purification efficiency, and in addition, ultimately, deliver the fusion protein into immunocytes efficiently and/or increase immunogenicity, by comprising a human thioredoxin protein in which cysteine residues are modified.

Other embodiment of the present application, relates to a vaccine composition or immunogenic composition comprising the fusion protein, nucleic acid molecule, expression vector, or cell.

Other embodiment of the present application, relates to a composition for preventing or treating cancer, infectious disease, autoimmune disease, or allergic disease, comprising the fusion protein, nucleic acid molecule, expression vector or cell.

The term, "immunogenic composition" means any composition which can induce an immune response. The term, "vaccine" refers to an immunogenic composition for reducing or preventing a risk of disease or infection, or improving or treating conventional disease or infection, by inducing an immune response.

These compositions may be a formulation in a form that contains the fusion protein according to the present application and is capable of being administered into a subject to cause an immune response. Therefore, the composition of the present invention may be conveniently used to prevent or improve or treat disease. When introduced into a subject or host, the composition may cause an immune response, and preferably, may cause a T-cell-mediated immune response.

The vaccine of the present invention may be an anti-cancer vaccine comprising a tumor antigen, for example, a tumor antigen sequence, particularly, neoantigen sequence, obtained from patient tumor analysis, and thereby, it may increase an anti-cancer immune effect by maximally triggering proliferation and activation of customized anti-cancer T cells.

The vaccine composition of the present application may also comprise an additional adjuvant that enhances effectiveness of the vaccine. The appropriate adjuvant includes (1) aluminum salts (alum), for example, aluminum hydroxide, aluminum phosphate, aluminum sulfate, etc.; (2) oil-in-water emulsion preparations (containing or not containing a specific immunostimulant such as muramyl peptide or bacterial cell wall components), for example, (a) MF59 comprising 5% squalene, 0.5% Tween 80 and 0.5% Span 85 (containing various amounts of N-acetylmuramyl-L-alanyl-D-isoglutaminyl-L-alanine-2-(1',2'-dipalmitoyl-sn-glycero-3-hydroxyphosphoryloxy)-ethylamine (MTP-PE), not necessarily, but selectively) formulated with submicron particles (WO90/14837), (b) SAF comprising 10% squalene, 0.4% Tween 80, 5% pluronic-blocked polymer and N-acetyl-muramyl-L-threonyl-D-isoglutamine (thr-MDP), shaken to be microfluidized with submicron particles or generate emulsion in a large particle size, and (c) RibiTM adjuvant system (RAS) comprising at least one bacterial cell wall component selected from the group consisting of monophosphoryl lipid A (MPL), trehalose dimycolate (TDM) and cell wall skeleton (CWS), 2% squalene, and 0.2% Tween 80; (3) saponin adjuvants; (4) complete adjuvants (CFA) and incomplete adjuvants (IFA) of Freund; (5) cytokines, for example, interleukins (for example, IL-1, IL-2, IL-4, IL-5, IL-6, IL-7, IL-12, etc.), interferons (for example, gamma interferon), macrophage colony-stimulating factors (M-CSF), tumor necrosis factors (TNF) and the like; (6) detoxified mutants of bacterial ADP-ribosylation toxins, for example, cholera toxins (CT), pertussis toxins (PT), or heat-labile toxins (LT) of E. coli, in particular, LT-R72, CT-S109, PT-K9/G129 (WO93/13302 and WO92/19265); and (7) other materials that act as an adjuvant that enhances effectiveness of a vaccine, but is not limited thereto.

The compositions of the present application may further comprise a pharmaceutically acceptable carrier, a diluent and/or an excipient in a commonly used amount if needed.

The pharmaceutically acceptable carrier is commonly used during formulation, and includes lactose, dextrose, sucrose, sorbitol, mannitol, starch, acacia gum, calcium phosphate, alginate, gelatin, calcium silicate, microcrystalline cellulose, polyvinyl pyrrolidone, cellulose, water, syrup, methyl cellulose, methylhydroxy benzoate, propylhydroxy benzoate, talc, magnesium stearate and mineral oil and the like, but is not limited thereto. The composition may further comprise a lubricant, a wetting agent, a sweetener, a flavoring agent, an emulsifier, a suspending agent, a preservative and the like, in addition to the components.

The fusion protein or composition comprising it as an active ingredient may be prepared in a unit dose form or be prepared by inserting it into a multi-dose container, by formulating using a pharmaceutically acceptable carrier and/or an excipient, according to the method that can be easily performed by those skilled in the art.

For example, the vaccine composition may comprise suspended or dissolved common salt water or buffered aqueous medium. For example, a diluent, for example, water, salt water, glycerol, ethanol and the like may be commonly used, and an auxiliary substance, for example, a wetting agent, an emulsifier, a pH buffer and the like may be present in the composition.

Forms suitable for injection include sterile aqueous solutions (aqueous) or dispersions for instant preparation of a sterile injection solution or dispersion and sterile powders. They should be stable under preparation conditions, and be preserved against microbial contamination of microorganisms such as bacteria or fungi. Microbial contamination may be prevented using various anti-bacterial agents and anti-fungal agents, for example, paraben, chlorobutanol, phenol, sorbic acid, thimerosal and the like. In many cases, it is preferable to comprise an isotonic agent, for example, sugar or sodium chloride. Prolonged absorption of the injection composition may be possible by using a drug which delays absorption, for example, aluminum monostearate or gelatin in the composition.

A sterile injection solution is prepared by combining various other components listed above with a required amount of the fusion protein in the afore-mentioned solvent, and filtration-sterilizing the fusion protein and/or other components other than heat-sensitive adjuvant cytokines and the like, for example, buffer solution such as PBS, if needed. In general, a dispersion is prepared by comprising various sterilized active ingredients into a sterile vehicle containing a basic dispersion medium and other components required from those described above. In case of sterile powders for preparing a sterile injection solution, a preferable method for preparation is vacuum drying and freeze-drying methods. By these techniques, powders of active ingredients, and any targeted components from the afore-mentioned sterile-filtered solutions are obtained.

The patient subjected to administration of the fusion protein and a composition comprising it as an active ingredient may be mammals, for example, primates such as humans, monkeys and the like, rodents including rats, mice, and the like, but not limited thereto.

Other aspect of the present invention relates to a method for causing, inducing and/or promoting an immune response against an antigen in a human, for example, cancer patient, and the method includes administering an effective amount of the vaccine composition or fusion protein described above into a patient, for example, cancer patient.

The term, "immune response" refers to a change in the activity of cells of the immune system, for example, B cells, T cells or monocytes, as a result of direct or indirect stimulation through cellular or cytokine mediation. The immune response may be specific (T cells and/or B cells) and/or non-specific immune responses.

Delivering the fusion protein according to the present invention, without limiting the action of the present invention in any way, is particularly useful for inducing an immune response, in particular, a T cell response, for example, a CD4+ T cell response or a CD8+ T cell response against an antigen. The CD4+ T cell response and CD8+ T cell response may occur together with or independently of a humoral response or other specific or non-specific immune response.

Other aspect of the present invention is related to use the fusion protein of the present invention regarding treatment and/or prevention of disease conditions. Examples of disease that can be treated according to the method of the present invention include various kinds of cancer diseases, infectious diseases, autoimmune diseases and allergic diseases.

For example, the cancer may be solid cancer or blood cancer, and non-restrictive examples may be breast cancer, lung cancer, prostate cancer, ovarian cancer, brain cancer, liver cancer, cervical cancer, endometrial cancer, uterine cancer, colon cancer, large intestine cancer, colorectal cancer, rectal cancer, kidney cancer, nephroblastoma, skin cancer, oral squamous epithelial cancer, epidermoid cancer, nasopharyngeal cancer, head and neck cancer, bone cancer, esophageal cancer, bladder cancer, lymphatic cancer (for example, Hodgkin lymphoma or non-Hodgkin lymphoma), gastric cancer, pancreatic cancer, testicular cancer, thyroid cancer, thyroid follicular cancer, melanoma, myeloma, multiple myeloma, mesothelioma, osteosarcoma, myelodysplastic syndrome, tumor of mesenchymal origin, soft tissue sarcoma, liposarcoma, gastrointestinal stromal sarcoma, malignant peripheral nerve sheath tumor (MPNST), Ewing sarcoma, leiomyosarcoma, mesenchymal chondrosarcoma, lymphosarcoma, fibrosarcoma, rhabdomyosarcoma, teratocarcinoma, neuroblastoma, medulloblastoma, glioma, skin benign tumor or leukemia. The lung cancer may be for example, small cell lung cancer (SCLC) or non-small cell lung cancer. The leukemia may be for example, acute myeloid leukemia (AML), chronic myeloid leukemia (CML), acute lymphoid leukemia (ALL) or chronic lymphoid leukemia (CLL). A subject to be treated may be a subject receiving secondary anti-hyperproliferative therapy. For example, the secondary anti-hyperproliferative therapy may be chemotherapy, radiotherapy, immunotherapy, phototherapy, cryotherapy, toxin therapy, hormone therapy, or surgery.

Therefore, other aspect of the present invention provides a method for preventing or treating disease, for example, cancer, infectious disease, autoimmune disease, or allergic disease, by enhancing an immune response using the composition. Herein, administration of the composition causes, induces or otherwise promotes an immune response that inhibits, stops, or delays occurrence or progression of disease conditions.

Direct delivery of the composition may be generally delivered systemically, subcutaneously, intradermally, intraperitoneally, intravascularly (intravenously), intramuscularly or locally, or is delivered into tissue spaces. The composition may also be administered into lesions. The regimen may be a single-dose or multiple-dose schedule.

The term, "effective dose" means an amount sufficient to achieve desired results, for example, an effective amount for treating or preventing cancer, when administered to a subject including humans. The effective dose may vary depending on factors such as the subject's disease condition, age, gender and body weight and the like. The dosage or treatment regimen may be adjusted to provide an optimal therapeutic response as understood by those skilled in the art.

The treatment regimen of a subject using a therapeutically effective dose may consist of single administration, or as another example, include a series of applications. The duration of the treatment period is determined depending on various factors such as the severity of disease, the age of the subject, the concentration of a vaccine, the patient's responsiveness to a vaccine or combinations thereof. In addition, it will be understood that the effective dose of the vaccine used for treatment may be increased or decreased during the course of an individual treatment regimen. Dosage may vary and it may be clarified by standard diagnostic assay known in the art. The vaccine of the present invention, for example, cancer vaccine, may be administered before treatment, during treatment, or after treatment using common anti-cancer agents, radiotherapy, hormone therapy, biotherapy and/or surgical tumor excision.

Other embodiment of the present application relates to a method for preparation of a fusion protein, comprising linking a human thioredoxin protein to the N-terminus, C-terminus or both of the peptide antigen, wherein at least 3 cysteine residues are substituted with non-cysteine residues in the amino acid sequence of the human thioredoxin protein.

In one embodiment, the linking may be performed *ex vivo* or *in vitro.*

In one embodiment, the fusion protein may have enhanced immunogenicity compared to a fusion protein to which a wild type human thioredoxin protein is linked.

In one embodiment, the fusion protein may have enhanced efficacy of preventing or treating disease, compared to a fusion protein to which a wild type human thioredoxin protein is linked.

In one embodiment, the fusion protein may have improved purification efficiency compared to a fusion protein to which a wild type human thioredoxin protein is linked.

In one embodiment, the linking a human thioredoxin protein to the N-terminus, C-terminus or both of the peptide antigen may be performed by a common chemical method or the recombinant method described above.

In one embodiment, the method for preparation of a fusion protein may additionally comprise preparing a human thioredoxin protein variant wherein at least 3 cysteine residues are substituted with non-cysteine residues in the amino acid sequence of the human thioredoxin protein before linking a human thioredoxin protein to the N-terminus, C-terminus or both of the peptide antigen.

The method for preparation of a fusion protein of the present application is a method for preparing the fusion protein according to one embodiment of the present application described above, and overlapping contents between both inventions are commonly applied also in the method for preparation of a fusion protein, and to avoid excessive complexity of the present description, description thereof is omitted.

Other embodiment of the present application, provides a method for enhancing immunogenicity of a fusion protein, comprising preparing a fusion protein by linking a human thioredoxin protein at the N-terminus, C-terminus or both of the peptide antigen, wherein at least 3 cysteine residues are substituted with non-cysteine residues in the amino acid sequence of the human thioredoxin protein.

Other embodiment, provides a method for enhancing efficacy of preventing or treating disease of a fusion protein, comprising preparing a fusion protein by linking a human thioredoxin protein at the N-terminus, C-terminus or both of the peptide antigen, wherein at least 3 cysteine residues are substituted with non-cysteine residues in the amino acid sequence of the human thioredoxin protein.

Other embodiment, provides a method for improving purification efficiency of a fusion protein, comprising preparing a fusion protein by linking a human thioredoxin protein into the N-terminus, C-terminus or both of the peptide antigen, wherein at least 3 cysteine residues are substituted with non-cysteine residues in the amino acid sequence of the human thioredoxin protein.

The method for enhancing immunogenicity, method for enhancing efficacy of preventing or treating disease and method for improving purification efficiency of the fusion protein of the present application comprise preparing the fusion protein according to one embodiment of the present application described above, and overlapping contents between both inventions are commonly applied, and to avoid excessive complexity of the present description, description thereof is omitted.

[BRIEF DESCRIPTION OF THE DRAWINGS]

FIG. 1 shows the results of confirming the peak shape after the second stage of column chromatography during the purification process of TrxA-M30-His (pc0735) according to one example of the present application.

FIG. 2 shows the results of confirming the peak shape after the second stage of column chromatography during the purification process of TrxA.v2-M30-His (pc0802) according to one example of the present application.

FIG. 3 shows the results of confirming the peak shape after the second stage of column chromatography during the purification process of hTrx.v2-M30-His (pc0763) according to one example of the present application.

FIG. 4 shows the results of confirming the peak shape after the second stage of column chromatography during the purification process of hTrx.v1-M30-His (pc0764) according to one example of the present application.

FIG. 5 shows the results of confirming the peak shape after the second stage of column chromatography during the purification process of hTrx.v3-M30-His (pc0792) according to one example of the present application.

FIG. 6 shows the results of confirming the peak shape after the second stage of column chromatography during the purification process of hTrx.v3-M30-PSBD-His (pc0890) according to one example of the present application.

FIG. 7 shows the results of confirming the tendency for serine residue substitution of a cysteine residue by comparing UV absorbance (280 nm) and area ratios over time.

FIG. 8 shows the results of confirming the peak shape after the second stage of column chromatography during the purification process of hTrx.v1-M44-His (pc0761) according to one example of the present application.

FIG. 9 shows the results of confirming the peak shape after the second stage of column chromatography during the purification process of hTrx.v2-M44-His (pc0762) according to one example of the present application.

FIG. 10 shows the results of confirming the peak shape after the second stage of column chromatography during the purification process of hTrx.v3-M44-PSBD-His (pc0895) according to one example of the present application.

FIG. 11 shows the results of confirming the peak shape after the second stage of column chromatography during the purification process of hTrx.v3-M12-His (pc0811) according to one example of the present application.

FIG. 12 shows the results of confirming the peak shape after the second stage of column chromatography during the purification process of hTrx.v3-M12-PSBD-His (pc0883) according to one example of the present application.

FIG. 13 shows the results of confirming the peak shape after the second stage of column chromatography during the purification process of hTrx.v3-M21-His (pc0812) according to one example of the present application.

FIG. 14 shows the results of confirming the peak shape after the second stage of column chromatography during the purification process of hTrx.v3-M21-PSBD-His (pc0869) according to one example of the present application.

FIG. 15 shows the results of confirming the peak shape after the second stage of column chromatography during the purification process of hTrx.v3-CT5-His (pc0824) according to one example of the present application.

FIG. 16 shows the results of confirming the peak shape after the second stage of column chromatography during the purification process of hTrx.v3-CT5-PSBD-His (pc0886) according to one example of the present application.

FIG. 17 shows the results of confirming the peak shape after the second stage of column chromatography during the purification process of hTrx.wt-MelanA-His (pc0993) according to one example of the present application.

FIG. 18 shows the results of confirming the peak shape after the second stage of column chromatography during the purification process of hTrx.v3- MelanA-His (pc0992) according to one example of the present application.

FIG. 19 shows the SDS-PAGE results of TrxA-M30-His (pc0735) according to one example of the present application.

FIG. 20 shows the SDS-PAGE results of hTrx.v2-M30-His (pc0763) according to one example of the present application.

FIG. 21 shows the SDS-PAGE analysis results of hTrx.v3-M30-His (pc0792) and TrxA.v2-M30-His (pc0802) according to one example of the present application.

FIG. 22 shows the SDS-PAGE analysis results of hTrx.v1-M44-His (pc0761) and hTrx.v2-M44-His (pc0762) according to one example of the present application.

FIG. 23 shows the SDS-PAGE analysis results of hTrx.v3-M44-PSBD-His (pc0895) according to one example of the present application.

FIG. 24 shows the SDS-PAGE analysis results of hTrx.v3-M12-His (pc0811) according to one example of the present application.

FIG. 25 shows the SDS-PAGE analysis results of hTrx.v3-M12-PSBD-His (pc0883) according to one example of the present application.

FIG. 26 shows the SDS-PAGE analysis results of hTrx.v3-M21-His (pc0812) according to one example of the present application.

FIG. 27 shows the SDS-PAGE analysis results of hTrx.v3-M21-PSBD-His (pc0869) according to one example of the present application.

FIG. 28 shows the SDS-PAGE analysis results of hTrx.v3-CT5-His (pc0824) according to one example of the present application.

FIG. 29 shows the SDS-PAGE analysis results of hTrx.v3-CT5-PSBD-His (pc0886) according to one example of the present application.

FIG. 30 shows the SDS-PAGE analysis results of hTrx.wt-MelanA-His (pc0993) according to one example of the present application.

FIG. 31 shows the SDS-PAGE analysis results of hTrx.v3- MelanA-His (pc0992) according to one example of the present application.

FIG. 32 shows the SDS-PAGE analysis results of hTrx.v3-MelanA-PSBD-His (pc0984) according to one example of the present application.

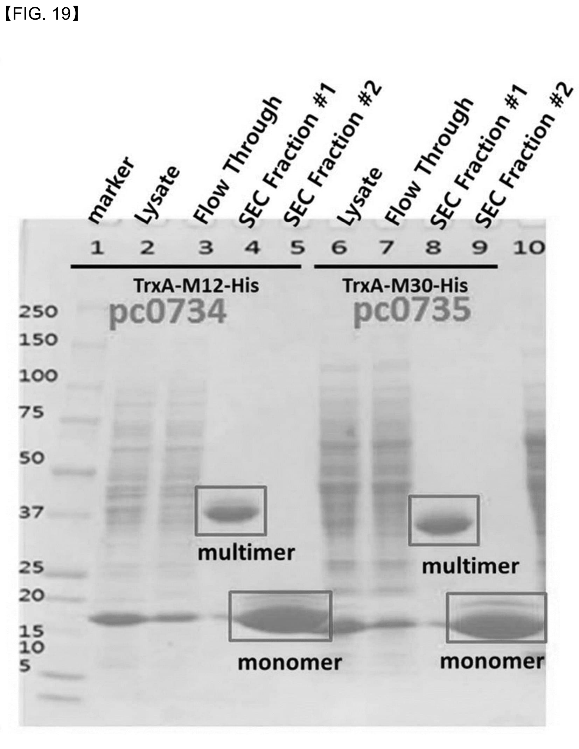

In FIG. 19 to FIG. 32, FT indicates Flow Through, and SEC # number indicates the number according to the fractionation order during Size-Exclusion Chromatography (SEC), and LB (loading before) indicates the start sample to be loaded onto the purification column.

[MODE FOR INVENTION]

Hereinafter, the present invention will be described in more detail by the following examples. However, they are intended to illustrate the contents of the present invention only, but the scope of the present invention is not limited by these examples.

Example 1. Selection of epitopes

Whole exome sequencing (WES) of cancer cells and normal cells, for identification of mutations of mice and selection of epitopes that bind to MHC, and mRNA sequencing for identification of expression of mutant genes were performed. By analysis of WES results, mutations that changed the protein sequence (missense mutation, frameshift mutation, insertion-deletion) and mouse MHC types were identified.

Specifically, as the normal cells, tail tissues of mice were used, and as the cancer cells, mouse melanoma cell line B16-F10 and mouse colorectal carcinoma cell lines MC38 and CT26 were used to perform next generation sequencing (NGS). NGS was performed by Macrogen, and Missense Single-nucleotide polymorphism (SNP) mutations were analyzed by WES with Mutect2 algorithm. Based on SNP, 13mer sequences were attached in the front and back, to find 27mer amino acids.

In the mRNA sequencing, only genes with an expression level, that is, an RPKM (Reads Per Kilobase Million) value of 10 or higher were selected from the WES sequencing results.

The epitope sequences derived from the results were input in public-domain programs of NetMHCCons and NetMHCPanll. The MHC type was input as a phenotype suitable for the mice, and Strong Binding (SB) and Weak Binding (WB) criteria were set as default. NetMHCCons was analyzed by 9mer each, and NetMHCPanll was analyzed by 13mer each. They were input in another program, IEBD Immunogenicity program, and they were analyzed by 9mer each and scores were input.

$$\text{Prediction Score} = 10 \times \text{number of NetMHCCons Binders}$$

+ number of NetMHCPanll Binders
+ IEBD Immunogenicity Score

The higher the prediction score, the higher the probability of showing efficacy, so the top 20 were selected and all of them were checked for immune responses, and among them, substances that showed immune responses or substances that were easy to prepare were selected and evaluation of anticancer efficacy was conducted. In addition, using these selected epitopes, fusion proteins for anticancer vaccines were prepared. The used neoantigens and tumor-associated antigens were shown in Table 2 below. In the table below, "CT+number" is arbitrary designation of the neoantigen number of the CT26 colorectal cancer cell line, and "M + number" is arbitrary designation of the neoantigen number of the melanoma cell line (B16-F10), and MelanA indicated MART-1, a tumor antigen found in melanoma.

[Table 2]

| Epitopes | | |
|---|---|---|
| SEQ ID NO: | Epitope name | Amino acid sequence |
| 1 | M30 | PSKPSFQEFVDWENVSPELNSTDQPFL |
| 2 | M44 | EFKHIKAFDRTFANNPGPMVVFATPGM |
| 3 | M12 | TPPPEEAMPFEFNGPAQGDHSQPPLQV |
| 4 | M21 | SSPDEVALVEGVQSLGFTYLRLKDNYM |
| 5 | CT5 | IYLESVAIMPQLFMVSK |
| 6 | MelanA | YPKKGHGHSYTTAEELAGIGILTVILG |

**Example 2. Selection of carrier proteins**

**2-1. Design of carrier proteins**

Neoantigens induce T-cell immunity by binding to MHC-I (Major Histocompatibility Complex-I) or MHC-II proteins. When Synthetic Long Peptides (SLPs) including neoantigens were used as antigens, in general, they are known to have a short half-life in the body and are known to have low solubility depending on the sequence. In order to complement these disadvantages of SLPs, an attempt was made to prepare a fusion protein in which a neoantigen and a thioredoxin protein are linked, and the fusion protein was expressed in E. coli and was to be produced through a purification process.

The fusion protein was expressed with an ORF (Open Reading Frame) consisting of (thioredoxin) - (linker 1) - (neoantigen) - (linker 2) - (His-tag); or (thioredoxin) - (linker 1) - (neoantigen) - (linker 2) - (carrier protein) - (linker 3) - (His-tag) in the direction from the N-terminus to the C-terminus. As physicochemical properties of the sequence of the expressed antigen are different, to purify antigens regardless of the antigen sequence characteristics, an affinity tag, His-tag (6 His) was arranged at the ORF-C terminus. The carrier protein was placed between the antigen and His-Tag so that any antigen sequence did not interfere with purification by the affinity tag, and the main components of the ORF were linked with a linker consisting of glycine and serine, and the used linkers were shown in Table 3 below. The types of the human thioredoxin and carrier proteins implemented in the invention are as follows. PSBD means a variant of a fragment of a full-length PSBD (SEQ ID NO: 13), and is the same as in the following examples.

[Table 3]

| SEQ ID NO: | Name | Amino acid sequence |
|---|---|---|
| | Amino acid sequences of thioredoxin proteins, carrier proteins, linkers and used fusion proteins (mutated amino acid residues are underlined) | |
| 7 | hTrx.v1 (human Thiore-doxin variant1) | MVKQIESKTAFQEALDAAGDKLVVVDFSATWCGPCKMIKPFFHSLSEKYSNVIFLEVDVDD**S**QDVASE**S**EVK**S**MPTFQFFKKGQKVGEFSGANKEKLEATINELV- Mutant (C62S, C69S, C73S) |
| 8 | hTrx.v2 (human Thiore-doxin variant2) | MVKQIESKTAFQEALDAAGDKLVVVDFSATWCGPCKMIKPFFHSLSEKYSNVIFLEVDVDDCQDVASECEVK**S**MPTFQFFKKGQKVGEFSGANKEKLEATINELV- Mutant (C73S) |
| 9 | hTrx.v3(human Thiore-doxin variant3) | MVKQIESKTAFQEALDAAGDKLVVVDFSATW**S**GP**S**KMIKPFFHSLSEKYSNVIFLEVDVDD**S**QDVASE**S**EVK**S**MPTFQFFKKGQKVGEFSGANKEKLEATINELV - Mutant (C32S, C35S, C62S, C69S, C73S) |
| 10 | hTrx.wt (human Thiore-doxin wildtype) | MVKQIESKTAFQEALDAAGDKLVVVDFSATWCGPCKMIKPFFHSLSEKYSNVIFLEVDVDDCQDVASECEVKCMPTFQFFKKGQKVGEFSGANKEKLEATINELV |

(continued)

| SEQ ID NO: | Name | Amino acid sequence |
|---|---|---|
| | Amino acid sequences of thioredoxin proteins, carrier proteins, linkers and used fusion proteins (mutated amino acid residues are underlined) | |
| 11 | TrxA(Escherichia coli, Thioredoxin 1) | MSDKIIHLTDDSFDTDVLKADGAILVDFWAEWCGP CKMIAPILDEIADEYQGKLTVAKLNIDQNPGTAPKY GIRGIPTLLLFKNGEVAATKVGALSKGQLKEFLDAN LA |
| 12 | TrxA.v2(Escherichia coli, Thioredoxin 1 variant 2) | MSDKIIHLTDDSFDTDVLKADGAILVDFWAEW**S**GP **S**KMIAPILDEIADEYQGKLTVAKLNIDQNPGTAPKY GIRGIPTLLLFKNGEVAATKVGALSKGQLKEFLDAN LA |
| 13 | PSBD(peripheral subunit-binding domain) | VIAMPSVRKYAREKGVDIRLVQGTGKNGRVLKEDI DA**W**LA |
| 14 | TrxA-M30 | MSDKIIHLTDDSFDTDVLKADGAILVDFWAEWCGP CKMIAPILDEIADEYQGKLTVAKLNIDQNPGTAPKY GIRGIPTLLLFKNGEVAATKVGALSKGQLKEFLDAN LAGGGGSPSKPSFQEFVDWENVSPELNSTDQPFL |
| 15 | hTrx.v2-M30 | MVKQIESKTAFQEALDAAGDKLVVVDFSATWCGP CKMIKPFFHSLSEKYSNVIFLEVDVDDCQDVASECE VKSMPTFQFFKKGQKVGEFSGANKEKLEATINELV GGGGSPSKPSFQEFVDWENVSPELNSTDQPFL |

(continued)

| SEQ ID NO: | Name | Amino acid sequence |
|---|---|---|
| | Amino acid sequences of thioredoxin proteins, carrier proteins, linkers and used fusion proteins (mutated amino acid residues are underlined) | |
| 16 | hTrx.v1-M30 | VKQIESKTAFQEALDAAGDKLVVVDFSATWCGPCK MIKPFFHSLSEKYSNVIFLEVDVDDSQDVASESEVK SMPTFQFFKKGQKVGEFSGANKEKLEATINELVGG GGSPSKPSFQEFVDWENVSPELNSTDQPFL |
| 17 | hTrx.v1-M30 | MVKQIESKTAFQEALDAAGDKLVVVDFSATWCGP CKMIKPFFHSLSEKYSNVIFLEVDVDDSQDVASESE VKSMPTFQFFKKGQKVGEFSGANKEKLEATINELV GGGGSPSKPSFQEFVDWENVSPELNSTDQPFL |
| 18 | hTrx.v3-M30 | MVKQIESKTAFQEALDAAGDKLVVVDFSATWSGPS KMIKPFFHSLSEKYSNVIFLEVDVDDSQDVASESEV KSMPTFQFFKKGQKVGEFSGANKEKLEATINELVG GGGSPSKPSFQEFVDWENVSPELNSTDQPFL |
| 19 | TrxA.v2-M30 | MSDKIIHLTDDSFDTDVLKADGAILVDFWAEWSGP SKMIAPILDEIADEYQGKLTVAKLNIDQNPGTAPKY GIRGIPTLLLFKNGEVAATKVGALSKGQLKEFLDAN LAGGGGSPSKPSFQEFVDWENVSPELNSTDQPFL |

(continued)

| SEQ ID NO: | Name | Amino acid sequence |
|---|---|---|
| | Amino acid sequences of thioredoxin proteins, carrier proteins, linkers and used fusion proteins (mutated amino acid residues are underlined) | |
| 20 | hTrx.v3-M30-PSBD | MVKQIESKTAFQEALDAAGDKLVVVDFSATWSGPS KMIKPFFHSLSEKYSNVIFLEVDVDDSQDVASESEV KSMPTFQFFKKGQKVGEFSGANKEKLEATINELVG GGGGPSKPSFQEFVDWENVSPELNSTDQPFLGG GGGVIAMPSVRKYAREKGVDIRLVQGTGKNGRVL KEDIDAWLA |
| 21 | hTrx.v1-M44 | MVKQIESKTAFQEALDAAGDKLVVVDFSATWCGP CKMIKPFFHSLSEKYSNVIFLEVDVDDSQDVASESE VKSMPTFQFFKKGQKVGEFSGANKEKLEATINELV GGGGSEFKHIKAFDRTFANNPGPMVVFATPGM |
| 22 | hTrx.v2-M44 | MVKQIESKTAFQEALDAAGDKLVVVDFSATWCGP CKMIKPFFHSLSEKYSNVIFLEVDVDDCQDVASECE VKSMPTFQFFKKGQKVGEFSGANKEKLEATINELV GGGGSEFKHIKAFDRTFANNPGPMVVFATPGM |
| 23 | hTrx.v3-M44-PSBD | MVKQIESKTAFQEALDAAGDKLVVVDFSATWSGPS KMIKPFFHSLSEKYSNVIFLEVDVDDSQDVASESEV KSMPTFQFFKKGQKVGEFSGANKEKLEATINELVG GGGGEFKHIKAFDRTFANNPGPMVVFATPGMGGG GGVIAMPSVRKYAREKGVDIRLVQGTGKNGRVLKE DIDAWLA |

(continued)

| SEQ ID NO: | Name | Amino acid sequence |
|---|---|---|
| | Amino acid sequences of thioredoxin proteins, carrier proteins, linkers and used fusion proteins (mutated amino acid residues are underlined) | |
| 24 | hTrx.v3-M12 | MVKQIESKTAFQEALDAAGDKLVVVDFSATWSGPS KMIKPFFHSLSEKYSNVIFLEVDVDDSQDVASESEV KSMPTFQFFKKGQKVGEFSGANKEKLEATINELVG GGGSTPPPEEAMPFEFNGPAQGDHSQPPLQV |
| 25 | hTrx.v3-M12-PSBD | MVKQIESKTAFQEALDAAGDKLVVVDFSATWSGPS KMIKPFFHSLSEKYSNVIFLEVDVDDSQDVASESEV KSMPTFQFFKKGQKVGEFSGANKEKLEATINELVG GGGGTPPPEEAMPFEFNGPAQGDHSQPPLQVGG GGGVIAMPSVRKYAREKGVDIRLVQGTGKNGRVL KEDIDAWLA |
| 26 | hTrx.v3-M21 | MVKQIESKTAFQEALDAAGDKLVVVDFSATWSGPS KMIKPFFHSLSEKYSNVIFLEVDVDDSQDVASESEV KSMPTFQFFKKGQKVGEFSGANKEKLEATINELVG GGGSSSPDEVALVEGVQSLGFTYLRLKDNYM |
| 27 | hTrx.v3-M21-PSBD | MVKQIESKTAFQEALDAAGDKLVVVDFSATWSGPS KMIKPFFHSLSEKYSNVIFLEVDVDDSQDVASESEV KSMPTFQFFKKGQKVGEFSGANKEKLEATINELVG GGGGSSPDEVALVEGVQSLGFTYLRLKDNYMGGG GGVIAMPSVRKYAREKGVDIRLVQGTGKNGRVLKE DIDAWLA |

(continued)

| SEQ ID NO: | Name | Amino acid sequence |
|---|---|---|
| | Amino acid sequences of thioredoxin proteins, carrier proteins, linkers and used fusion proteins (mutated amino acid residues are underlined) | |
| 28 | hTrx.v3-CT5 | MVKQIESKTAFQEALDAAGDKLVVVDFSATWSGPSKMIKPFFHSLSEKYSNVIFLEVDVDDSQDVASESEVKSMPTFQFFKKGQKVGEFSGANKEKLEATINELVGGGGSIYLESVAIMPQLFMVSK |
| 29 | hTrx.v3-CT5-PSBD | MVKQIESKTAFQEALDAAGDKLVVVDFSATWSGPSKMIKPFFHSLSEKYSNVIFLEVDVDDSQDVASESEVKSMPTFQFFKKGQKVGEFSGANKEKLEATINELVGGGGGIYLESVAIMPQLFMVSKGGGGGVIAMPSVRKYAREKGVDIRLVQGTGKNGRVLKEDIDAWLA |
| 30 | hTrx.wt-MelanA | MVKQIESKTAFQEALDAAGDKLVVVDFSATWCGPCKMIKPFFHSLSEKYSNVIFLEVDVDDCQDVASECEVKCMPTFQFFKKGQKVGEFSGANKEKLEATINELVGGGGGYPKKGHGHSYTTAEELAGIGILTVILG |
| 31 | hTrx.v3-MelanA | MVKQIESKTAFQEALDAAGDKLVVVDFSATWSGPSKMIKPFFHSLSEKYSNVIFLEVDVDDSQDVASESEVKSMPTFQFFKKGQKVGEFSGANKEKLEATINELVGGGGGYPKKGHGHSYTTAEELAGIGILTVILG |

(continued)

| SEQ ID NO: | Name | Amino acid sequence |
|---|---|---|
| | Amino acid sequences of thioredoxin proteins, carrier proteins, linkers and used fusion proteins (mutated amino acid residues are underlined) | |
| 32 | hTrx.v3-MelanA-PSBD | MVKQIESKTAFQEALDAAGDKLVVVDFSATWSGPS KMIKPFFHSLSEKYSNVIFLEVDVDDSQDVASESEV KSMPTFQFFKKGQKVGEFSGANKEKLEATINELVG GGGGYPKKGHGHSYTTAEELAGIGILTVILGGGGG GVIAMPSVRKYAREKGVDIRLVQGTGKNGRVLKED IDAWLA |
| 33 | pc0735 (TrxA-M30-His) | MSDKIIHLTDDSFDTDVLKADGAILVDFWAEWCGP CKMIAPILDEIADEYQGKLTVAKLNIDQNPGTAPKY GIRGIPTLLLFKNGEVAATKVGALSKGQLKEFLDAN LAGGGGSPSKPSFQEFVDWENVSPELNSTDQPFL SSGGHHHHHH |
| 34 | pc0763 (hTrx.v2-M30-His) | MVKQIESKTAFQEALDAAGDKLVVVDFSATWCGP CKMIKPFFHSLSEKYSNVIFLEVDVDDCQDVASECE VKSMPTFQFFKKGQKVGEFSGANKEKLEATINELV GGGGSPSKPSFQEFVDWENVSPELNSTDQPFLGG HHHHHH |

(continued)

| SEQ ID NO: | Name | Amino acid sequence |
|---|---|---|
| | Amino acid sequences of thioredoxin proteins, carrier proteins, linkers and used fusion proteins (mutated amino acid residues are underlined) | |
| 35 | pc0764 (hTrx.v1-M30-His) | MVKQIESKTAFQEALDAAGDKLVVVDFSATWCGPCKMIKPFFHSLSEKYSNVIFLEVDVDDSQDVASESEVKSMPTFQFFKKGQKVGEFSGANKEKLEATINELVGGGGSPSKPSFQEFVDWENVSPELNSTDQPFLGGHHHHHH |
| 36 | pc0792 (hTrx.v3-M30-His) | MVKQIESKTAFQEALDAAGDKLVVVDFSATWSGPSKMIKPFFHSLSEKYSNVIFLEVDVDDSQDVASESEVKSMPTFQFFKKGQKVGEFSGANKEKLEATINELVGGGSPSKPSFQEFVDWENVSPELNSTDQPFLGGHHHHH |
| 37 | pc0802 (TrxA.v2-M30-His) | MSDKIIHLTDDSFDTDVLKADGAILVDFWAEWSGPSKMIAPILDEIADEYQGKLTVAKLNIDQNPGTAPKYGIRGIPTLLLFKNGEVAATKVGALSKGQLKEFLDANLAGGGGSPSKPSFQEFVDWENVSPELNSTDQPFLGGHHHHHH |

(continued)

| SEQ ID NO: | Name | Amino acid sequence |
|---|---|---|
| 38 | pc0890 (hTrx.v3-M30-PSBD-His) | MVKQIESKTAFQEALDAAGDKLVVVDFSATWSGPS KMIKPFFHSLSEKYSNVIFLEVDVDDSQDVASESEV KSMPTFQFFKKGQKVGEFSGANKEKLEATINELVG GGGGPSKPSFQEFVDWENVSPELNSTDQPFLGG GGGVIAMPSVRKYAREKGVDIRLVQGTGKNGRVL KEDIDAWLAGGHHHHHH |
| 39 | pc0761 (hTrx.v1-M44-His) | MVKQIESKTAFQEALDAAGDKLVVVDFSATWCGP CKMIKPFFHSLSEKYSNVIFLEVDVDDSQDVASESE VKSMPTFQFFKKGQKVGEFSGANKEKLEATINELV GGGGSEFKHIKAFDRTFANNPGPMVVFATPGMSS GGHHHHHH |
| 40 | pc0762 (hTrx.v2-M44-His) | MVKQIESKTAFQEALDAAGDKLVVVDFSATWCGP CKMIKPFFHSLSEKYSNVIFLEVDVDDCQDVASECE VKSMPTFQFFKKGQKVGEFSGANKEKLEATINELV GGGGSEFKHIKAFDRTFANNPGPMVVFATPGMSS GGHHHHHH |

(continued)

| SEQ ID NO: | Name | Amino acid sequence |
|---|---|---|
| | Amino acid sequences of thioredoxin proteins, carrier proteins, linkers and used fusion proteins (mutated amino acid residues are underlined) | |
| 41 | pc0895 (hTrx.v3-M44-PSBD-His) | MVKQIESKTAFQEALDAAGDKLVVVDFSATWSGPS KMIKPFFHSLSEKYSNVIFLEVDVDDSQDVASESEV KSMPTFQFFKKGQKVGEFSGANKEKLEATINELVG GGGGEFKHIKAFDRTFANNPGPMVVFATPGMGGG GGVIAMPSVRKYAREKGVDIRLVQGTGKNGRVLKE DIDAWLAGGHHHHHH |
| 42 | pc0811(hTrx.v3-M12-His) | MVKQIESKTAFQEALDAAGDKLVVVDFSATWSGPS KMIKPFFHSLSEKYSNVIFLEVDVDDSQDVASESEV KSMPTFQFFKKGQKVGEFSGANKEKLEATINELVG GGGSTPPPEEAMPFEFNGPAQGDHSQPPLQVGG HHHHHH |
| 43 | pc0883 (hTrx.v3-M12-PSBD-His) | MVKQIESKTAFQEALDAAGDKLVVVDFSATWSGPS KMIKPFFHSLSEKYSNVIFLEVDVDDSQDVASESEV KSMPTFQFFKKGQKVGEFSGANKEKLEATINELVG GGGGTPPPEEAMPFEFNGPAQGDHSQPPLQVGG GGVIAMPSVRKYAREKGVDIRLVQGTGKNGRVL KEDIDAWLAGGHHHHHH |

(continued)

| SEQ ID NO: | Name | Amino acid sequence |
|---|---|---|
| | Amino acid sequences of thioredoxin proteins, carrier proteins, linkers and used fusion proteins (mutated amino acid residues are underlined) | |
| 44 | pc0812 (hTrx.v3-M21-His) | MVKQIESKTAFQEALDAAGDKLVVVDFSATWSGPS KMIKPFFHSLSEKYSNVIFLEVDVDDSQDVASESEV KSMPTFQFFKKGQKVGEFSGANKEKLEATINELVG GGGSSSPDEVALVEGVQSLGFTYLRLKDNYMGGH HHHHH |
| 45 | pc0869 (hTrx.v3-M21-PSBD-His) | MVKQIESKTAFQEALDAAGDKLVVVDFSATWSGPS KMIKPFFHSLSEKYSNVIFLEVDVDDSQDVASESEV KSMPTFQFFKKGQKVGEFSGANKEKLEATINELVG GGGGSSPDEVALVEGVQSLGFTYLRLKDNYMGGG GGVIAMPSVRKYAREKGVDIRLVQGTGKNGRVLKE DIDAWLAGGHHHHHH |
| 46 | pc0824 (hTrx.v3-CT5-His) | MVKQIESKTAFQEALDAAGDKLVVVDFSATWSGPS KMIKPFFHSLSEKYSNVIFLEVDVDDSQDVASESEV KSMPTFQFFKKGQKVGEFSGANKEKLEATINELVG GGGSIYLESVAIMPQLFMVSKGGHHHHHH |

(continued)

| SEQ ID NO: | Name | Amino acid sequence |
|---|---|---|
| | Amino acid sequences of thioredoxin proteins, carrier proteins, linkers and used fusion proteins (mutated amino acid residues are underlined) | |
| 47 | pc0886 (hTrx.v3-CT5-PSBD-His) | MVKQIESKTAFQEALDAAGDKLVVVDFSATWSGPS KMIKPFFHSLSEKYSNVIFLEVDVDDSQDVASESEV KSMPTFQFFKKGQKVGEFSGANKEKLEATINELVG GGGGIYLESVAIMPQLFMVSKGGGGGVIAMPSVRK YAREKGVDIRLVQGTGKNGRVLKEDIDAWLAGGH HHHHH |
| 48 | pc0993 (hTrx.wt-MelanA-His) | MVKQIESKTAFQEALDAAGDKLVVVDFSATWCGP CKMIKPFFHSLSEKYSNVIFLEVDVDDCQDVASECE VKCMPTFQFFKKGQKVGEFSGANKEKLEATINELV GGGGGYPKKGHGHSYTTAEELAGIGILTVILGGGH HHHH |
| 49 | pc0992 (hTrx.v3-Mela-nA-His) | MVKQIESKTAFQEALDAAGDKLVVVDFSATWSGPS KMIKPFFHSLSEKYSNVIFLEVDVDDSQDVASESEV KSMPTFQFFKKGQKVGEFSGANKEKLEATINELVG GGGGYPKKGHGHSYTTAEELAGIGILTVILGGGHH HHHH |

(continued)

| SEQ ID NO: | Name | Amino acid sequence |
|---|---|---|
| | Amino acid sequences of thioredoxin proteins, carrier proteins, linkers and used fusion proteins (mutated amino acid residues are underlined) | |
| 50 | pc0984 (hTrx.v3-Mela-nA-PSBD-His) | MVKQIESKTAFQEALDAAGDKLVVVDFSATWSGPS KMIKPFFHSLSEKYSNVIFLEVDVDDSQDVASESEV KSMPTFQFFKKGQKVGEFSGANKEKLEATINELVG GGGGYPKKGHGHSYTTAEELAGIGILTVILGGGGG GVIAMPSVRKYAREKGVDIRLVQGTGKNGRVLKED IDAWLAGGHHHHHH |
| 51 | GS linker 1 | GGGGS |
| 52 | GS linker 2 | SSGG |
| 53 | GS linker 3 | GG |
| 54 | GS linker 4 | GGGGG |

## 2-2. Results of preparations of neoantigen fusion proteins by linked protein

Since protein sequences of neoantigens of anti-cancer vaccines are different from each other, even if they are used with carrier proteins, the pI and hydrophobicity vary depending on the neoantigen protein sequence, so there is a difficulty to purify all antigens by Ion Exchange Chromatography or Hydrophobic Interaction Chromatography. For this reason, it was attempted to purify antigen proteins using Affinity Chromatography, and purification was performed by selecting His-tag with a small tag size of 6 histidine amino acids and a high Protein Binding Capacity of 20 mg per Resin 1 mL as an affinity tag. The antigens primarily purified with the affinity tag was polished using secondary Size-Exclusion Chromatography (SEC). The cloning, culture and purification methods are the same as Example 3. The results of preparation of neoantigen fusion proteins in which each carrier protein was combined were as follows.

[Table 4]

| | | | | Results of preparation of neoantigen fusion proteins by linked protein | | |
|---|---|---|---|---|---|---|
| N-terminus | C-terminus | Neoant igen | Lot No | Construction | Prepared antigen concentration (mg/mL) | Amount of prepara tion (mg) |
| TrxA | - | M30 | pc0735 | TrxA-M30-His | 4.1 | 47.6 |
| TrxA.v2 | - | | pc0802 | TrxA.v2-M30-His | 4.05 | 20.3 |
| hTrx.v1 | - | | pc0764 | hTrx.v1-M30-His | 2.37 | 22.7 |
| hTrx.v2 | - | | pc0763 | hTrx.v2-M30-His | 2.23 | 20.5 |
| hTrx.v3 | - | | pc0792 | hTrx.v3-M30-His | 3.79 | 13.6 |
| hTrx.v3 | PSBD | | pc0890 | hTrx.v3-M30-PSBD-His | 2.27 | 21.5 |
| hTrx.v1 | - | M44 | Pc0761 | hTrx.v1-M44-His | 2.49 | 19.4 |
| hTrx.v2 | - | | Pc0762 | hTrx.v2-M44-His | 4.62 | 48.1 |
| hTrx.v3 | PSBD | | pc0895 | hTrx.v3-M44-PSBD-His | 3.11 | 32.1 |

(continued)

| Results of preparation of neoantigen fusion proteins by linked protein | | | | | | |
|---|---|---|---|---|---|---|
| N-terminus | C-terminus | Neoant igen | Lot No | Construction | Prepared antigen concentration (mg/mL) | Amount of prepara tion (mg) |
| hTrx.v3 | - | M12 | pc0811 | hTrx.v3-M12-His | 5.09 | 61.1 |
| hTrx.v3 | PSBD | | pc0883 | hTrx.v3-M12-PSBD-His | 3.58 | 36.9 |
| hTrx.v3 | - | M21 | Pc0812 | hTrx.v3-M21-His | 2.00 | 19.0 |
| hTrx.v3 | PSBD | | pc0869 | hTrx.v3-M21-PSBD-His | 4.84 | 62.9 |
| hTrx.v3 | - | CT5 | pc0824 | hTrx.v3-CT5-His | 2.43 | 21.9 |
| hTrx.v3 | PSBD | | pc0886 | hTrx.v3-CT5-PSBD-His | 2.29 | 20.8 |
| hTrx.wt | - | Melan | pc0993 | hTrx.wt-MelanA-His | 0.733 | 3.5 |
| hTrx.v3 | - | A | pc0992 | hTrx.v3-MelanA-His | 1.03 | 7.1 |
| hTrx.v3 | PSBD | | Pc0984 | hTrx.v3-MelanA-PSBD-His | 2.71 | 28.4 |

## Example 3. Preparation and culture of vectors

### 3-1. Construction of vectors

In case that the length of Insert was short (within 400mer) when a fusion protein was synthesized, oligos of 60-90mer of no more than 6 were obtained, and then overlap PCR was performed, and in case that the length was long, through gene synthesis, sequences with restriction enzyme sites at the front were obtained. Primers and backbone sequences required for construction of each construct were summarized in Table 5 and Table 6. After treating the corresponding sequences and backbone vectors with restriction enzymes for 1 hour, and then gel extraction was performed, and the obtained gene fragment was treated with ligase for 1 hour, and then *E. coli* Transformation was performed.

The fusion protein sequence consists of a thioredoxin protein, a neoantigen, a carrier protein which may be further comprised, and a tag sequence for purification. They were constructed so as to insert them into a vector after producing all sequences to be produced through overlap PCR or gene synthesis, or insert a desired neoantigen sequence easily through oligo synthesis by adding a restriction enzyme site separately before and after the position to where the neoantigen was to be inserted, after determining the sequence and order of the fusion protein, as the selected epitope sequence could continuously change according to the sequence analysis of the cancer type. In order to insert an additional sequence such as neoantigens and the like, they were constructed by performing cloning using them as an insert, after attaching two single sequences (two single-stranded oligo sequences of which a part is attached complementarily to each other) through an annealing process, and when the additional sequence became longer, they were produced by increasing the number of inserts to several numbers.

In case of PCR, primers and templates, polymerase premix and DW were added, and it was performed by denaturation at 95 degrees for 1 minutes, 25 cycles at 95°C for 10 seconds, at 60°C for 10 seconds, and at 72°C for 30 seconds, and then extension at 72°C for 2 minutes, and finally, at 12°C for 5 minutes in a PCR machine. Gene fragments obtained by performing gel extraction after treating the corresponding sequences and backbone vectors with restriction enzymes for 1 hour were treated with ligase for 1 hour, and then *E. coli* Transformation was performed. When Insert was constructed by oligo annealing, two oligo sequences attaching complementarily to each other were mixed at the same concentration of 1uM, and incubated for 10 minutes after setting to 95°C for 5 minutes, ramp rate 0.1°C/sec and lowering to 50°C.

The vector backbone produced first was derived from pET28a(+), and in case of pET28a(+), to change the restriction enzyme site, a pET-Backbone vector capable of being used as an enzyme site different from conventional ones by conducting PCR using a pET-Duet vector as a template and backbone F1 and backbone R1 primers, and then inserting fragments cleaved using restriction enzymes XbaI and XhoI sites into pET-28a(+). The vectors used in the experiment were produced based on this pET-Backbone vector. TrxA-M30-His was constructed by conducting PCR using the pET32a vector as a template and TrxA F SpeI and TrxA BamHI R as primers, and cleaving hCSTA-M30-His with NdeI-BamHI using the vector and ligating, and Trx.A2-M30-his was constructed by cutting what were subjected to overlap PCR using one as the forward fragment for which PCR was performed using the TrxA sequence as a template with TrxA F Spel and TrxA R SS mut, and one as the backward fragment for which PCR was performed using TrxA F SS mut primer and TrxA BamHI R primer, with Ndel-BamHI using TrxA-M30-His as the vector, and ligating. In addition, hTrx.v1-M30-His, and hTrx.v1-M44-His were constructed by performing PCR using the synthesized hTrx sequence as a template and hT F Ndel and hT R BamHI as primers, and cutting with Ndel-BamHI using TrxA-M30-His and TrxA-M44-His as vectors and ligating, and hTrx.v2-M30-His and hTrx.v2-M44-His were constructed by cutting what were subjected to overlap PCR using one as the forward fragment for which PCR was performed using the hTrx sequence as a template and hT F Ndel and 6269C 2frag F as primers, and one as the backward fragment for which PCR was performed using the same template and 6269C 1frag R and hT R BamHI as primers, with Ndel-BamHI using TrxA-M30-His and TrxA-M44-His as vectors, and ligating, and hTrx.wt-MelanA-His was constructed by cutting what were subjected to overlap PCR using one as the forward fragment for which PCR was performed using the hTrx.v2-M30-His as a template and T7 F and hTrx wt R as primers, and one as the backward fragment for which PCR was performed using hTrx.v3-melanA-PSBD-His as a template and hTrx wt F and MelanA wt R as primers, with Bglll-Sacl using hTrx.v3-MelanA-PSBD-His as a vector, and ligating.

hTrx.v3–neoantigen (M30, M44, M12, M21, or CT5)-His was constructed by cutting what were subjected to overlap PCR using one as the forward fragment for which PCR was performed using the hTrx sequence as a template and hT F Ndel and hTrx F SS mut as primers, and one as the backward fragment for which PCR was performed with hTrx R SS mut and hT R BamHI, with Bglll-Sacl using hTrx.v3-MelanA-PSBD-His as a vector, and ligating.

In order to construct a vector in the form of hTrx.v3-neoantigen-PSBD-His, it was constructed by constructing a backbone vector designed to insert a neoantigen using two Bsal sites positioned between hTrx.v3 and PSBD in the order of hTrx-Bsal enzyme site-PSBD-His and then inserting the neoantigen. For constructing the vector, overlap PCR was performed. Overlap PCR was performed using one as the first template for which PCR was conducted using the sequence of hTrx.v1 synthesized in Cosmogenetech as a template and hT Ndel F and hTrx.v3 R as primers, and one as the second template for which PCR was conducted using the preceding hTrx.v1 sequence as a template and hTrx.v3 F and hT Bsal R1 as primers, with the preceding hTrx.v3 F and hT Bsal R as primers, and the overlap PCR was conducted using the corresponding overlap PCR product as the first template again, and one as the second template for which PCR was performed using the PSBD sequence for which gene synthesis was performed in Cosmogenetech as a template with Bsal F and PSBD R, and using the corresponding previous PCR product forward primer, hT Ndel F and the reverse primer of the backward PCR product, PSBD R as primers, and this was cut with Ndel-Sacl and ligated into the backbone vectors made based on pET-28 and pET-Duet, respectively, thereby constructing the hTrx-Bsal enzyme site-PSBD-His vector. Herein, in order to produce hTrx.v3-neoantigen-PSBD-His, the hTrx-Bsal-PSBD-His vector was cut with Bsal and two F/R nucleotide oligomers according to each neoantigen (those to which the same neoantigen name is attached in the primer SEQ ID Nos: 48-49, 58-77) were attached through an annealing process, and then ligated with the vector cut previously, thereby constructing it.

As Comp.cell for transformation, DH5alpha (Enzynomics) was used, and selection was performed by applying heat shock for 1 minute and 30 seconds, and stabilizing in LB media for 1 hour, and then spreading in a solid medium containing an antibiotic marker. In media kept at 37 degrees overnight (12-16h), 1-2 colonies were grown in 2ml LB media for 6-8 hours, and then mini-prep was performed to obtain the vector, and then a certain amount was used for sequence analysis.

[Table 5]

| Backbone sequences | | |
|---|---|---|
| Backbone sequences | | |
| SED ID NO: | Carrier protein | Nucleotide sequence (5'→3') |
| 55 | hTrx.v1 | ATGGTTAAACAAATTGAGTCGAAAACCG CCTTCCAAGAAGCTCTTGATGCTGCTGG TGATAAGTTGGTCGTAGTTGACTTCTCA GCCACTTGGTGCGGTCCATGCAAAATG ATAAAGCCTTTTTTCCACTCGTTGAGTG AGAAGTATTCAAACGTAATTTTTTTAGAA GTGGATGTCGATGATTCACAAGATGTTG CAAGTGAGAGCGAAGTCAAGTCTATGC CCACGTTTCAATTTTTCAAAAAAGGTCA GAAGGTGGGCGAGTTTTCAGGTGCTAA TAAAGAAAAATTAGAAGCTACGATTAAT GAACTGGTC |

(continued)

| Backbone sequences | | |
|---|---|---|
| Backbone sequences | | |
| SED ID NO: | Carrier protein | Nucleotide sequence (5'→3') |
| 56 | hTrx.v2 | ATGGTTAAACAAATTGAGTCGAAAACCG CCTTCCAAGAAGCTCTTGATGCTGCTGG TGATAAGTTGGTCGTAGTTGACTTCTCA GCCACTTGGTGCGGTCCATGCAAAATG ATAAAGCCTTTTTTCCACTCGTTGAGTG AGAAGTATTCAAACGTAATTTTTTTAGAA GTGGATGTCGATGATTGCCAAGATGTTG CAAGTGAGTGCGAAGTCAAGTCTATGCC CACGTTTCAATTTTTCAAAAAAGGTCAGA AGGTGGGCGAGTTTTCAGGTGCTAATAA AGAAAAATTAGAAGCTACGATTAATGAA CTGGTC |

(continued)

| Backbone sequences | | |
|---|---|---|
| Backbone sequences | | |
| SED ID NO: | Carrier protein | Nucleotide sequence (5'→3') |
| 57 | hTrx.v3 | ATGGTTAAACAAATTGAGTCGAAAACCG CCTTCCAAGAAGCTCTTGATGCTGCTGG TGATAAGTTGGTCGTAGTTGACTTCTCA GCCACTTGGTCTGGTCCATCTAAAATGA TAAAGCCTTTTTTCCACTCGTTGAGTGA GAAGTATTCAAACGTAATTTTTTTAGAAG TGGATGTCGATGATTCACAAGATGTTGC AAGTGAGAGCGAAGTCAAGTCTATGCC CACGTTTCAATTTTTCAAAAAAGGTCAGA AGGTGGGCGAGTTTTCAGGTGCTAATAA AGAAAAATTAGAAGCTACGATTAATGAA CTGGTC |

(continued)

| Backbone sequences | | |
|---|---|---|
| Backbone sequences | | |
| SED ID NO: | Carrier protein | Nucleotide sequence (5'→3') |
| 58 | TrxA | ATGAGCGATAAAATTATTCACCTGACTG ACGACAGTTTTGACACGGATGTACTCAA AGCGGACGGGGCGATCCTCGTCGATTT CTGGGCAGAGTGGTGCGGTCCGTGCAA AATGATCGCCCCGATTCTGGATGAAATC GCTGACGAATATCAGGGCAAACTGACC GTTGCAAAACTGAACATCGATCAAAACC CTGGCACTGCGCCGAAATATGGCATCC GTGGTATCCCGACTCTGCTGCTGTTCAA AAACGGTGAAGTGGCGGCAACCAAAGT GGGTGCACTGTCTAAAGGTCAGTTGAAA GAGTTCCTCGACGCTAACCTGGCC |

(continued)

| Backbone sequences | | |
|---|---|---|
| Backbone sequences | | |
| SED ID NO: | Carrier protein | Nucleotide sequence (5'→3') |
| 59 | TrxA.v2 | ATGAGCGATAAAATTATTCACCTGACTG ACGACAGTTTTGACACGGATGTACTCAA AGCGGACGGGGCGATCCTCGTCGATTT CTGGGCAGAGTGGTCTGGTCCGTCTAA AATGATCGCCCCGATTCTGGATGAAATC GCTGACGAATATCAGGGCAAACTGACC GTTGCAAAACTGAACATCGATCAAAACC CTGGCACTGCGCCGAAATATGGCATCC GTGGTATCCCGACTCTGCTGCTGTTCAA AAACGGTGAAGTGGCGGCAACCAAAGT GGGTGCACTGTCTAAAGGTCAGTTGAAA GAGTTCCTCGACGCTAACCTGGCC |
| 60 | PSBD | GTTATCGCTATGCCGTCCGTACGCAAAT ATGCACGTGAAAAaGGCGTTGATATCCG CCTGGTTCAGGGTACCGGTAAAAACGG CCGTGTTCTGAAAGAAGACATCGACGC GTGGCTGGCT |

[Table 6]

| SEQ ID NO: | Primer name | Nucleotide sequence (5' →3') |
|---|---|---|
| | Primer sequence | |
| | Primer sequences | |
| 61 | backbone F1 | GCG*TCTAGA*AATAATTTTGTTTAACTTTAAGAAGGAGATATACATATGGCAGATCTCAATTGGAT |
| 62 | backbone R1 | CAGCGGTTTCTTTACCAGACTCGAG |
| 63 | TrxA F Spel | GCGACTAGTAATAATTTTGTTTAACTTTAAGAAGGAGATATACATATGAGCGATAAAA |
| 64 | TrxA F SS mut | TGGTCTGGTCCGTCTAAAATGATCGCCCCGATTCTGGAT |
| 65 | TrxA R SS mut | CATTTTAGACGGACCAGACCACTCTGCCCAGAAATCGACG |
| 66 | TrxA BamHI R | GCGGGATCCACCACCACCGGCCAGGTTAGCGTC |
| 67 | M30 F | AGGACCGAGCAAACCGTCGTTTCAGGAATTTGTGGACTGGGAAAACGTGTCGCCGGAACTGAACAGCACCGATCAGCCGTTTCTG |
| 68 | M30 R | CACCCAGAAACGGCTGATCGGTGCTGTTCAGTTCCGGCGACACGTTTTCCCAGTCCACAAATTCCTGAAACGACGGTTTGCTCGG |
| 69 | PSBD F | GCAAATATGCACGTGAAAAGGCGTTGATATCCGCCTGGTTCAGGGTACCGGTAAAAACGGCCGTGTTCTGAAAGAAGACATCGACGC |
| 70 | PSBD R | CGAC*GAGCTC*TCAATGGTGGTGATGGTGATGTCCGCCAGCCAGCCACGCGTCGATGTCTTCTTTCAGAACACGG |
| 71 | hT Ndel F | GCG*CATATG*GTTAAACAAATTGAGTCGAAAAC |
| 72 | hTrx.v3 R | TATCATTTTAGATGGACCAGACCAAGTGGCTGAGAAGTCAACTACGAC |
| 73 | hTrx.v3 F | CACTTGGTCTGGTCCATCTAAAATGATAAAGCCTTTTTTCCACTCGTTGAG |

(continued)

| | Primer sequence | | |
|---|---|---|---|
| | Primer sequences | | |
| | SEQ ID NO: | Primer name | Nucleotide sequence (5' →3') |
| | 74 | hT Bsal R1 | TGAGACCCAGCGTTGCGGGTCTCTTCCTCCACCACCAC CGACCAGTT |
| | 75 | hT Bsal R2 | CCGGTACCCTGAACCAGGCGGATATCAACGCCTTTTTCA CGTGCATATTTGCGTACGGACGGCATAGCGATAACTCCT CCACCACCACC |
| | 76 | Bsal F | agagaccCGCAACGCTGggtctcaGGTGGTGGTGgaGgaGTTA TCGCT |
| | 77 | M44 F | AGGAGAATTTAAACATATTAAAGCGTTTGATCGTACCTTT GCGAATAACCCGGGTCCGATGGTGGTGTTTGCGACCCC GGGTATG |
| | 78 | M44 R | CACCCATACCCGGGGTCGCAAACACCACCATCGGACCC GGGTTATTCGCAAAGGTACGATCAAACGCTTTAATATGTT TAAATTC |
| | 79 | M12 F | AGGAACCCCGCCGCCGGAAGAAGCGATGCCGTTTGAAT TTAATGGTCCGGCGCAGGGTGATCATAGCCAGCCGCCG CTGCAGGTG |
| | 80 | M12 R | CACCCACCTGCAGCGGCGGCTGGCTATGATCACCCTGC GCCGGACCATTAAATTCAAACGGCATCGCTTCTTCCGGC GGCGGGGT |

(continued)

| SEQ ID NO: | Primer name | Nucleotide sequence (5' →3') |
|---|---|---|
| | Primer sequence | |
| | Primer sequences | |
| 81 | M21 F | AGGAAGCAGCCCGGATGAAGTGGCGCTGGTTGAAGGTGTGCAGAGCCTGGGTTTTACCTATCTGCGTCTGAAAGATAATTATATG |
| 82 | M21 R | CACCCATATAATTATCTTTCAGACGCAGATAGGTAAAACCCAGGCTCTGCACACCTTCAACCAGCGCCACTTCATCCGGGCTGCT |
| 83 | CT5 F | AGGAATTTATCTGGAAAGCGTGGCGATTATGCCGCAGCTGTTTATGGTGAGCAAA |
| 84 | CT5 R | CACCTTTGCTCACCATAAACAGCTGCGGCATAATCGCCACGCTTTCCAGATAAAT |
| 85 | MelanA F | aggaTATCCGAAAAAGGCCATGGCCATAGCTATACCACCGCGGAAGAACTGGCGGGCATTGGCATTCTGACCGTGATTCTGGGC |
| 86 | MelanA R | CACCGCCCAGAATCACGGTCAGAATGCCAATGCCCGCCAGTTCTTCCGCGGTGGTATAGCTATGGCCATGGCCTTTTTCGGATA |
| 87 | T7 F | GTCCGGCGTAGAGGATCGAGATCTC |
| 88 | MelanA wt R | GCGGAGCTCTCAATGGTGGTGATGGTGATGTCCGCCTTCTTTCGCAATTTTGTTTTTCGCGCG |
| 89 | hTrx wt F | TGCGAAGTCAAGtgcATGCCCACGTTTCAATTTTTCAAAAAAGGTCAG |
| 90 | hTrx wt R | TGAAACGTGGGCATgcACTTGACTTCGCACTCACTTGCAACATC |

### 3-2. Culture

For the vectors of which sequences were confirmed, transformation was performed into ClearColi BL21 (DE3) for culture. Since comp.cell of the corresponding strain used electroporation, selection was performed by adding DNA, and then placing in a separate cuvette, and applying electric shock, and stabilizing in LB media for 1 hour, and then spreading in solid media containing an antibiotic marker. 1 colony grown in media kept at 37°C overnight (16-20h) was inoculated into LB media containing 3ml antibiotics, and was grown overnight, and then, in the morning of the next day, secondary inoculation was performed in 0.8 L media. When the OD grew to about 0.7-1.2, IPTG 0.2mM induction was performed and the culture temperature was lowered from 37°C → 25°C and culturing was conducted overnight, and the next day, cells were recovered.

### 3-3. Recovery and lysis of microbial cells

The cultured solution in which the recombinant fusion protein was expressed was placed in a 1L bottle for centrifuge, and then centrifuge was performed at 7000 rpm, 5±3°C for 5 minutes, and the supernatant was discarded and the precipitated microbial cells were recovered. The microbial cells recovered after the centrifuge were suspended with a lysis solution (20 mM Sodium phosphate, 0.3M NaCl, 10 mM Imidazole, 1mM PMSF pH8.0), and then the cells were lysed using a ultrasonicator. After lysing, the cell lysed solution was placed in a 50 mL bottle for centrifuge, and then centrifuge was performed at 40,000xg, 5±3°C for 30 minutes, and the supernatant was purified by Akta pure system.

### Example 4. Purification

#### 4-1. Purification of fusion proteins

Purification of fusion proteins were conducted using filtration and 2-step column chromatography.

Step 1: Metal ion affinity chromatography (IMAC)

Using 20 mM sodium phosphate / 0.3M sodium chloride / 10 mM imidazole buffer, a metal ion affinity column was equilibrated. After lysing, the centrifuged supernatant was filtered through a 0.22$\mu$m filter, and then loaded on the column, and washed with 20 mM sodium phosphate / 0.3M sodium chloride / 10 mM imidazole buffer (pH8.0). Subsequently, using 20 mM sodium phosphate / 0.3M sodium chloride / 150 mM imidazole buffer (pH8.0), recombinant anti-cancer vaccines were eluted.

Step 2: Size exclusion chromatography (SEC)

Using phosphate buffered physiological saline solution, a size exclusion chromatography column (Hiload Superdex G75) was equilibrated. The eluate eluted from the metal ion affinity column was loaded on the column, and then the ultraviolet absorbance (280 nm) was monitored and peak fractions of 50 mAU or more were collected.

#### 4-2. Analysis of chromatogram results

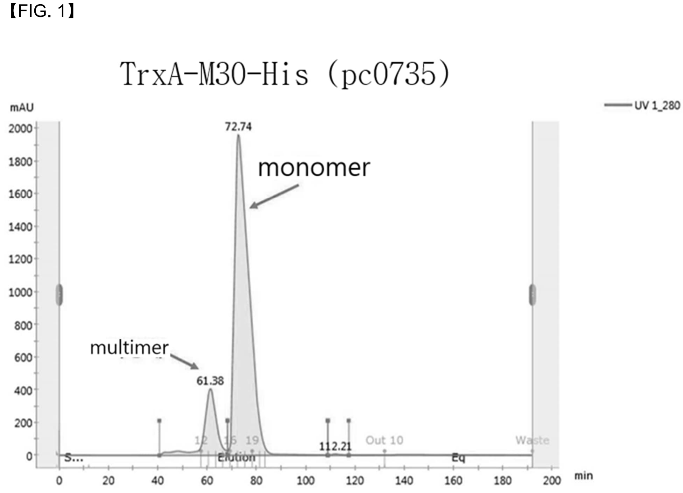

The forms of the multimer and monomer peaks depending on the variant were confirmed by analyzing SEC process chromatograms depending on each carrier protein variant (FIG. 1 to FIG. 18).

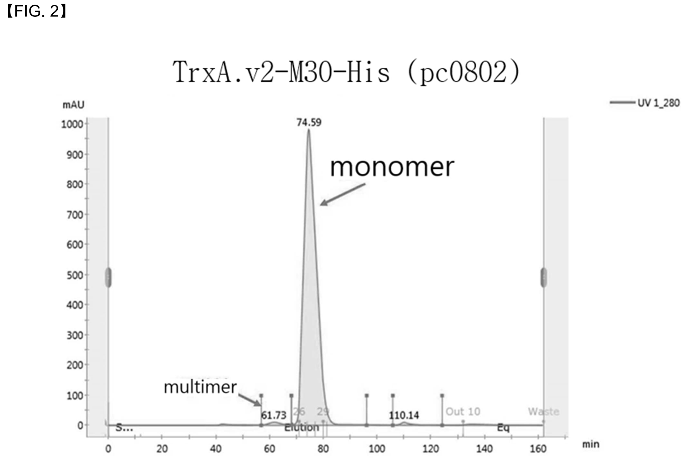

FIG. 1 is the chromatogram result of TrxA-M30-His (pc0735, wild type), and FIG. 2 is the chromatogram result of TrxA.v2-M30-His (pc0802, 2 Cys substitutions). When comparing the chromatograms of FIG. 1 and FIG. 2, it was confirmed that the peak of Retention Time corresponding to the multimer of wild type TrxA (TrxA-M30-His, pc0745) was not observed in the TrxA variant in which cysteine residues were substituted (TrxA.v2-M30-His, pc802), and thus, it could be seen that the purification efficiency of the variant was higher.

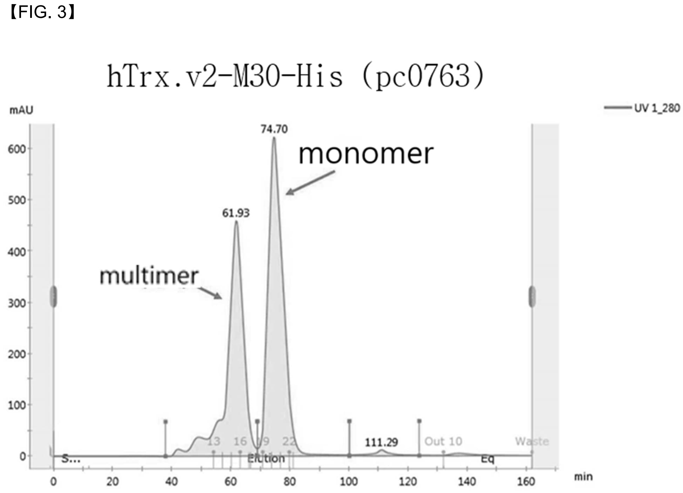

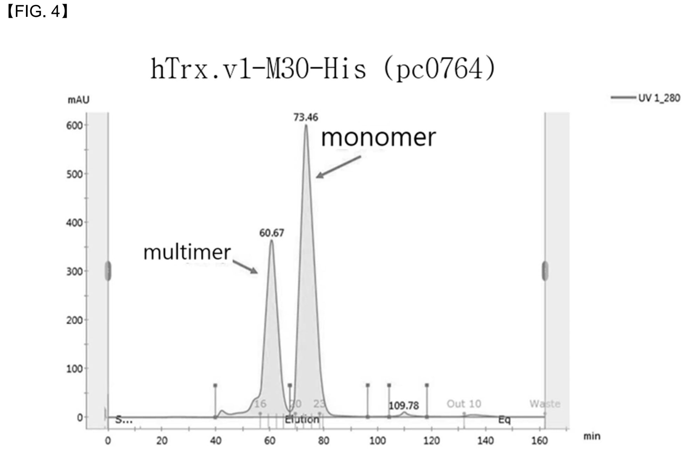

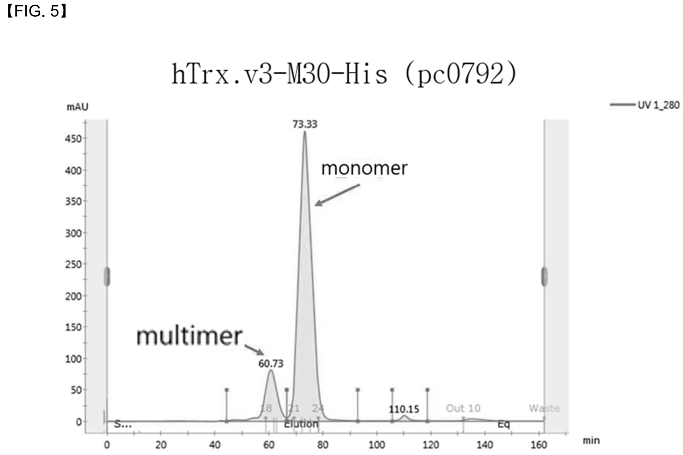

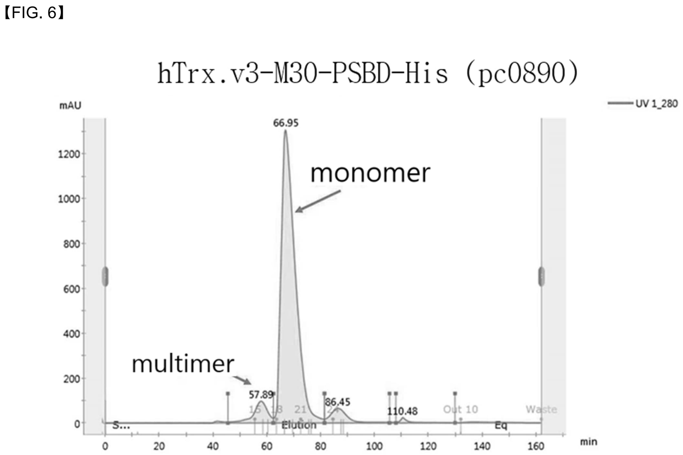

FIG. 3 is the chromatogram result of hTrx.v2-M30-His (pc0763, 1 Cys substitution), and FIG. 4 is the chromatogram result of hTrx.v1-M30-His (pc0764, 3 Cys substitutions), and FIG. 5 is the chromatogram result of hTrx.v3-M30-His (pc0792, 5 Cys 5 substitution), and FIG. 6 is the chromatogram result of hTrx.v3-M30-PSBD-His (pc0890, 5 Cys substitutions, further comprised carrier protein PSBD). As in FIG. 3 to FIG. 6, it was confirmed that the peak ratio of the multimer (peak #1) and monomer (peak #2) in the size exclusion chromatography process was shown differently according to the number of substitutions of hTrx cysteine residues and whether the carrier protein PSBD was further comprised. Consequently, it was found that the ratio of the monomer peak was shown the highest, as the cysteine residues present in the wild type were substituted with serine residues, and when PSBD was comprised at the C-terminus, the monomer peak ratio became higher.

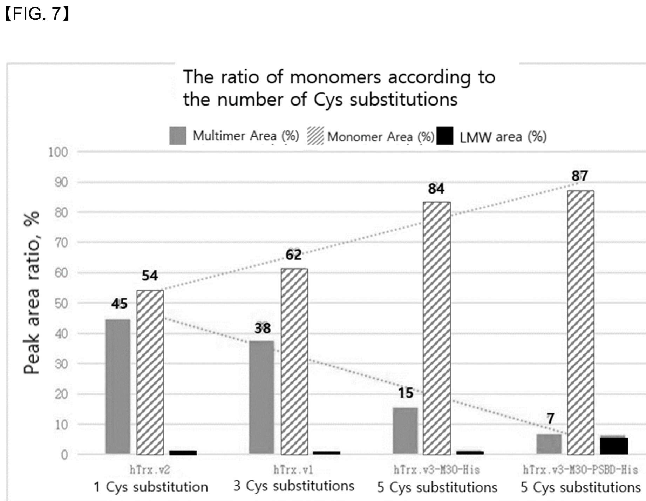

In addition, the tendency of cysteine residues for serine residue substitution was confirmed by comparing the area ratio depending on the ultraviolet absorbance (280nm) and time (FIG. 7). As a result of quantifying and comparing the monomer peak area depending on the number of substitutions of cysteine residues, it was confirmed that it was 54% in case of 1 substitution, and it was 84~87%, when 5 were substituted all. As such, it was shown that the structural stability of the monomer increased, as the number of substitutions of cysteine residues increased, and it was found that the structural stability of the monomer was further increased, when the carrier protein, PSBD was further comprised.

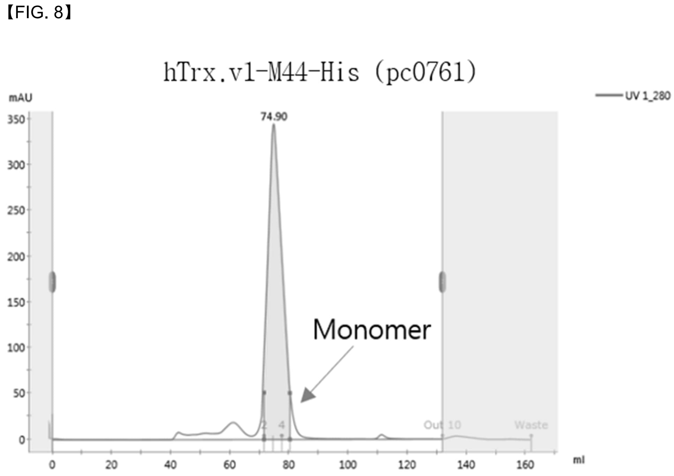

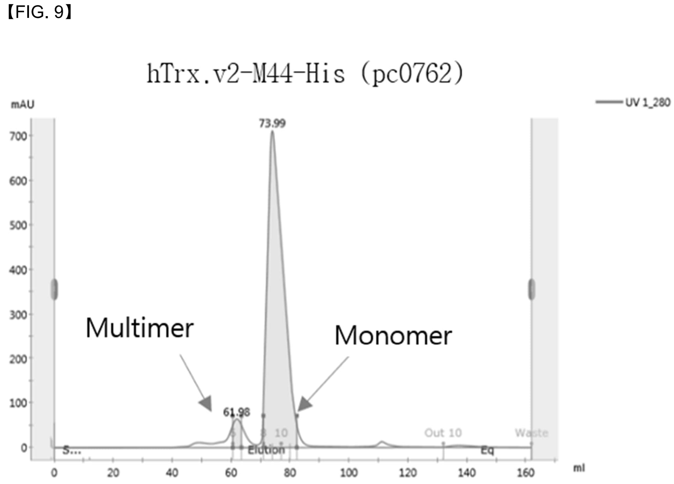

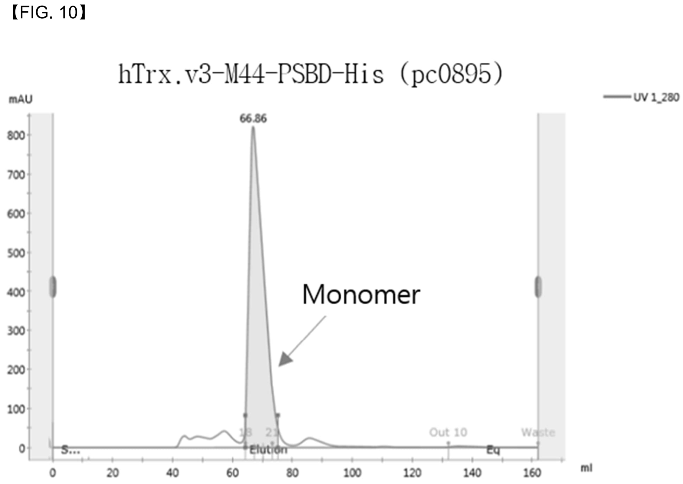

FIG. 8 is the chromatogram result of hTrx.v1-M44-His (pc0761, 3 Cys substitutions), and FIG. 9 is that of hTrx.v2-M44-His (pc0762, 1 Cys substitution), and FIG. 10 is that of hTrx.v3-M44-PSBD-His (pc0895, 5 Cys substitutions, comprising further comprised carrier protein PSBD). As shown in FIG. 8 to FIG. 10, it could be confirmed that the multimer peak was reduced and therefore, the purification efficiency increased, when the number of substitutions of cysteine residues of hTrx with serine residues was high, or PSBD was further comprised.

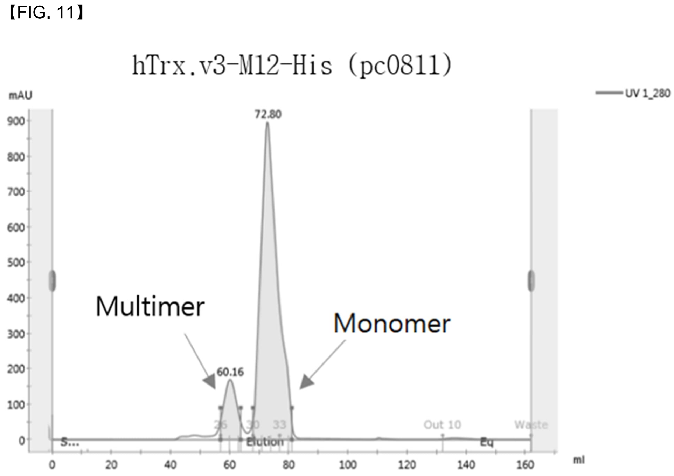

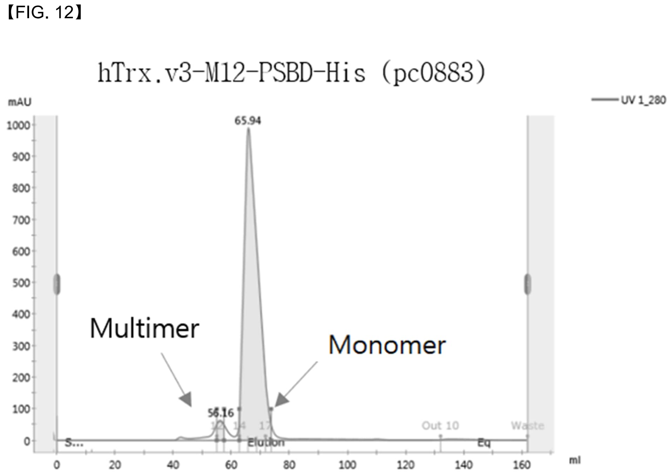

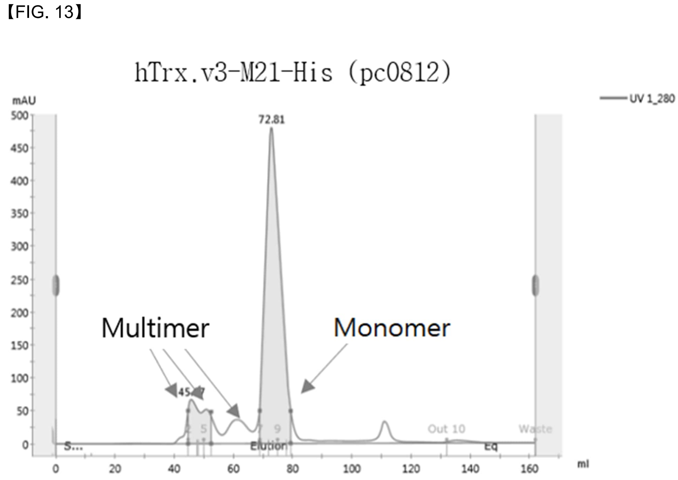

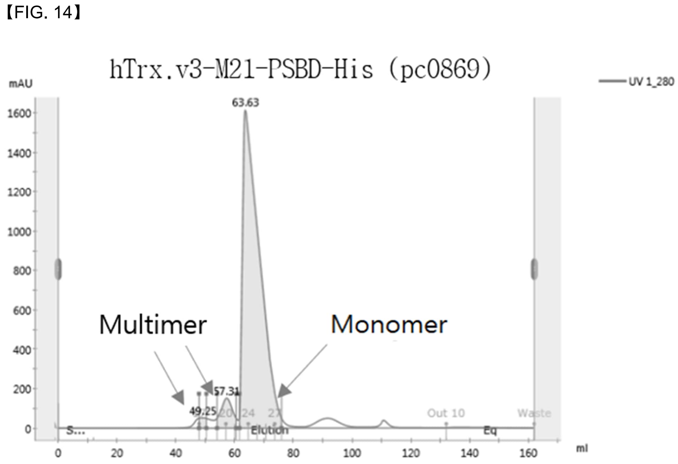

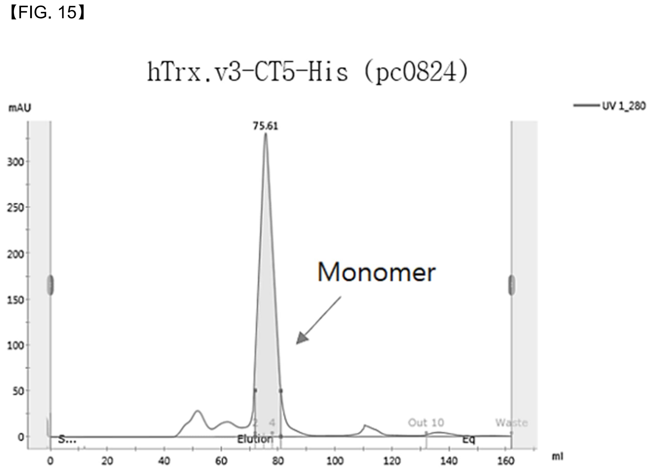

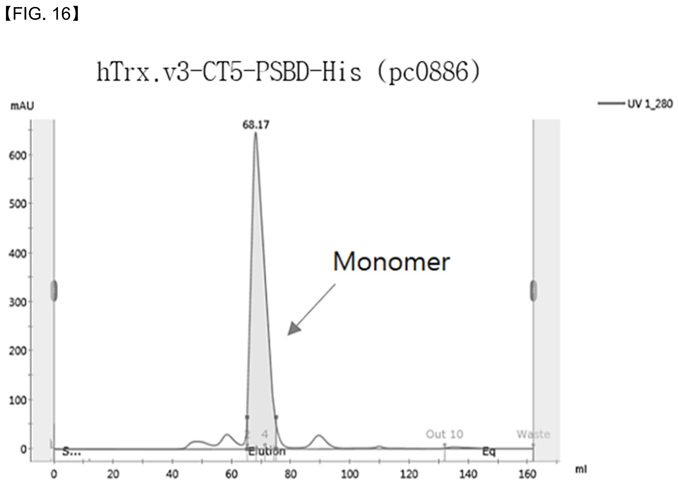

FIG. 11 is the chromatogram result of hTrx.v3-M12-His (pc0811, 5 Cys substitutions), and FIG. 12 is that of hTrx.v3-M12-PSBD-His (pc0883, 5 Cys substitutions, comprising further comprised carrier protein PSBD), and FIG. 13 is the chromatogram result of hTrx.v3-M21-His (pc0812, 5 Cys substitutions), and FIG. 14 is that of hTrx.v3-M21-PSBD-His (pc0869, 5 Cys substitutions, comprising further comprised carrier protein PSBD), and FIG. 15 is the chromatogram result of hTrx.v3-CT5-His (pc0824, 5 Cys substitutions), and FIG. 16 is that of hTrx.v3-CT5-PSBD-His (pc0886, 5 Cys 5 substitutions, comprising further comprised carrier protein PSBD). As shown in FIG. 11 to FIG. 16, it could be confirmed that the multimer peak was reduced and the size of the monomer peak was increased, and therefore, the purification efficiency of the fusion protein was increased, when the additional carrier protein, PSBD was comprised, regardless of the type of antigen.

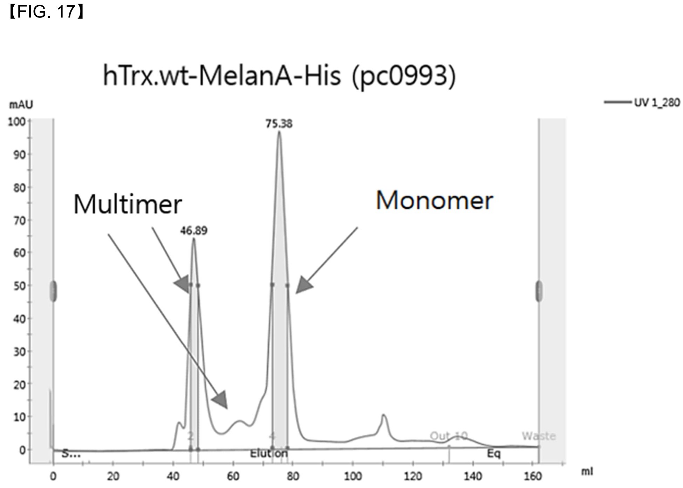

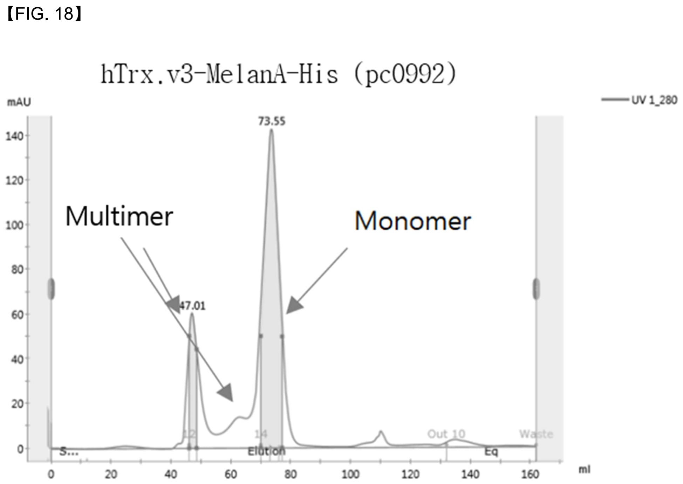

FIG. 17 is the chromatogram result of hTrx.wt-MelanA-His (pc0993, wild type), and FIG. 18 is that of hTrx.v3-MelanA-His (pc0992, 5 Cys substitutions). As shown in FIG. 17 and FIG. 18, it could be found that the multimer peak was reduced, and thus the purification efficiency of the fusion protein was increased, when the cysteine residues of hTrx was substituted with serine residues.

**4-3. Analysis of confirmation test of In process control**

On the other hand, a confirmation test (SDS-PAGE), and protein content (UV method) were conducted by In process control (IPC), and the results were shown in FIG. 19 to FIG. 32 and Table 4.

The confirmation test (SDS-PAGE) was performed by the following method. While preparing electrophoresis, first, the temperature of Heating Block (Cat No. Iso-Block) was adjusted to 95°C. Samples, distilled water (DW), Loading Dye (3X with Betamercaptoethanol) were mixed in an appropriate ration, and the mixed sample to be electrophoresed was boiled in the Heating Block heated to 95°C for 5 minutes. While boiling, Gel Tank was assembled with Tris-Glycine-PAG,SDS Gel (Cat No.: KG75355). SDS Solution was poured into the assembled Gel Tank, and connected to Electrophoresis Power Supply (Cat No.: EPS 1001) to warm up to 90V. After cooling the sample in which boiling was completed, Spin down was conducted using Microcentrifuge (13000 rpm, 1 min). To determine the protein size in SDS-Polyacrylamide Gel Well, Protein Marker (Precision Plus Protein Dual Color Standards. Cat No.: #161-0394 or EzWayTM Protein-PreBlue Ladder, Cat No.: #K18010) of about 5 uL was injected, and the sample of about 15 uL was injected. The power of Electrophoresis Power Supply was adjusted to 90 V - 20 mins, and the end time was adjusted to approximately 200 V - 1 hr. The SDS-Gel after electrophoresis was poured with an appropriate amount of Coomassie protein gel stain solution, and spun for under a condition of 70 rpm in ORBITAL SHAKER (Cat No.: NB-101S) 30 minutes or more. After gel staining, Coomassie protein gel stain solution was removed, and then spun with Fixing Solution for under a condition of 70 rpm in ORBITAL SHAKER (Cat No.: NB-101S), and the SDS-Gel without CBG staining was photographed and saved using Image Lab 6.0 program of BIO-RAD Gel Doc EZ Imager.

Protein content (UV method) was performed by the following method. A Path Length 1cm cuvette (Quartz) was clearly washed with distilled water, and the moisture was removed with a tissue that generates little particles such as Kimtex Wipes, and blown with an air gun. 200 uL of Reference substance (water for injection or PBS) was placed in a cuvette, and the absorbance at 280 nm was measured using a UV/Visible Spectrophotometer. After washing by repeating the processes of washing with distilled water, removing moisture, and blowing with the air gun, 200 uL of the sample was placed in a cuvette, and the absorbance at 280 nm was measured using a UV/Visible Spectrophotometer. The content was calculated with the value multiplied by the dilution factor of the sample and then divided by the Abs. 0.1% Coefficient constant that varies depending on the Protein Sequence.

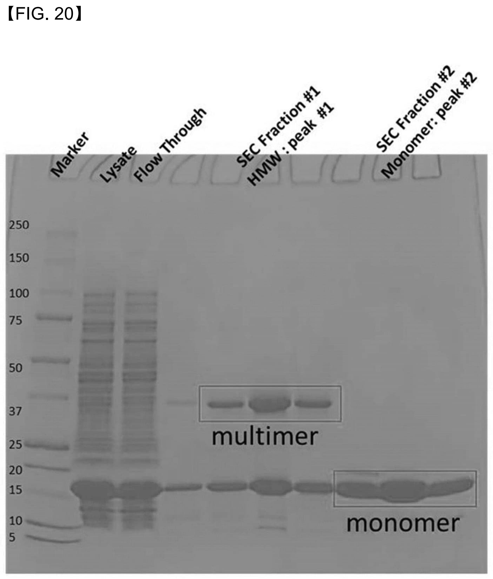

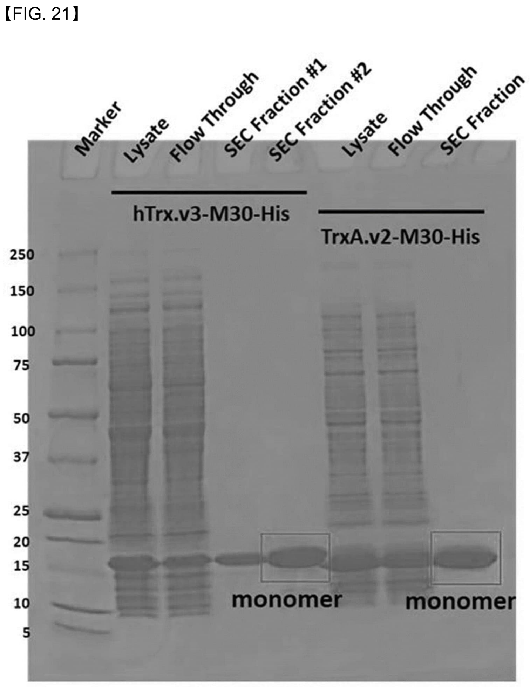

As the result of the reducing SDS-PAGE of the peak fractions corresponding to the multimers, the results that they were not completely released into monomers were confirmed, despite the treatment of a reducing agent. This was commonly shown in TrxA (wild type, Lane 6 to Lane 9) and hTrx.v2 (1 cysteine substitution) (FIG. 19 and FIG. 20). On the other hand, in case of TrxA.v2 and hTrx.v3 in which all the cysteine residues were substituted, as the result of the reducing SDS-PAGE analysis, no band corresponding to the multimers was confirmed (FIG. 21).

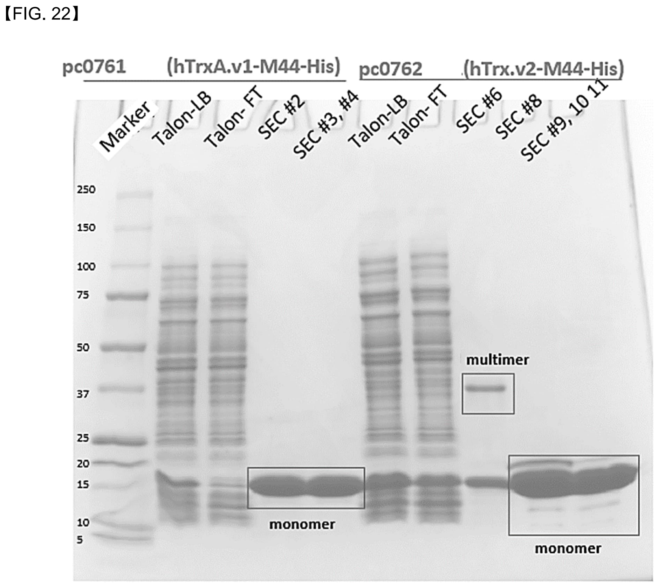

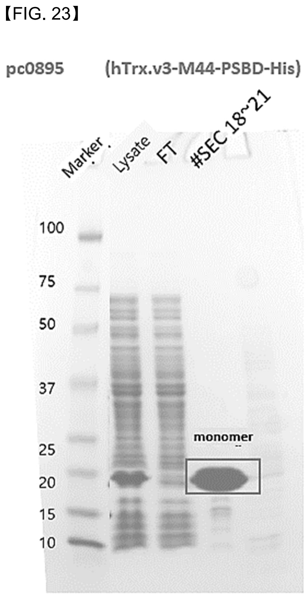

FIG. 22 shows the SDS-PAGE results of hTrx.v1-M44-His (pc0761, 3 Cys substitutions) and hTrx.v2-M44-His (pc0762, 1 Cys substitution), and FIG. 23 shows the SDS-PAGE results of hTrx.v3-M44-PSBD-His (pc0895, 5 Cys substitutions, comprising further comprised carrier protein PSBD). The multimer band was confirmed in hTrx.v2-M44-His (pc0762, 1 Cys substitution), but it was not confirmed in hTrx.v1-M44-His (pc0761, 3 Cys substitutions) and hTrx.v3-M44-PSBD-His (pc0895, 5 Cys substitutions, comprising further comprised carrier protein PSBD), and in particular, in case of hTrx.v3-M44-PSBD-His (pc0895, 5 Cys substitutions, comprising further comprised carrier protein PSBD), the monomer band was shown thicker, so it could be confirmed that the higher the number of cysteine substitutions and the more PSBD was comprised, generation of multimer was reduced, and therefore, the purification efficiency of the fusion protein was improved.

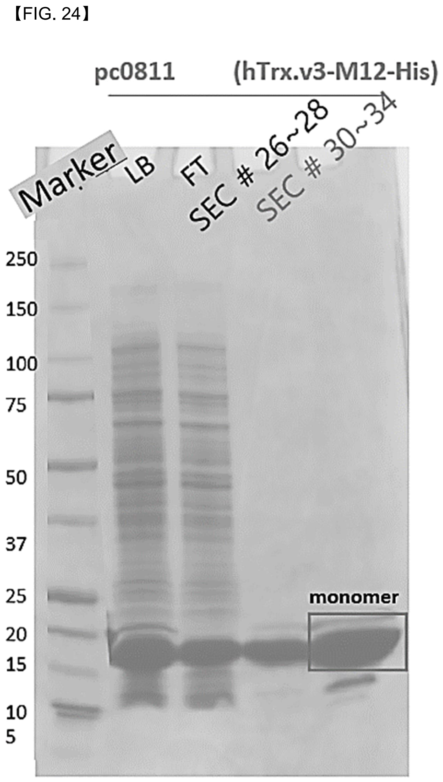

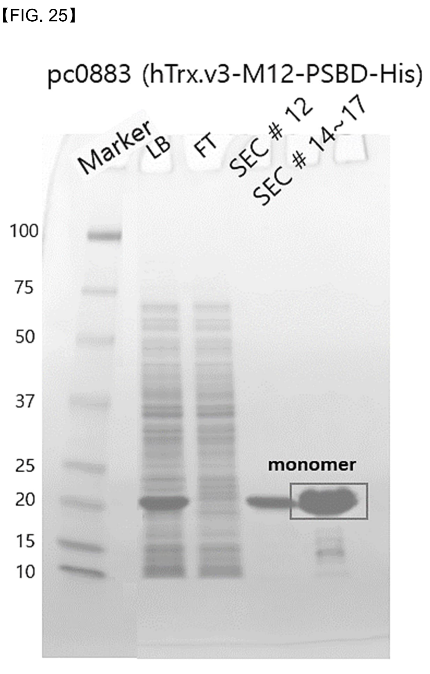

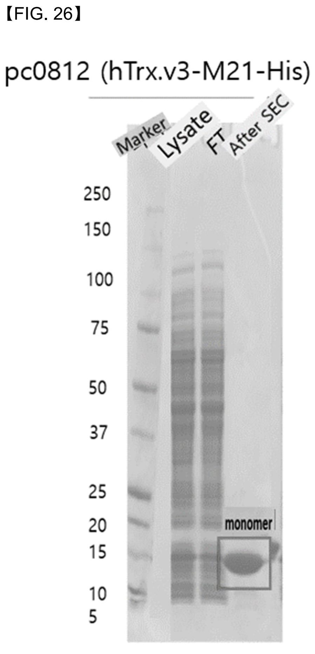

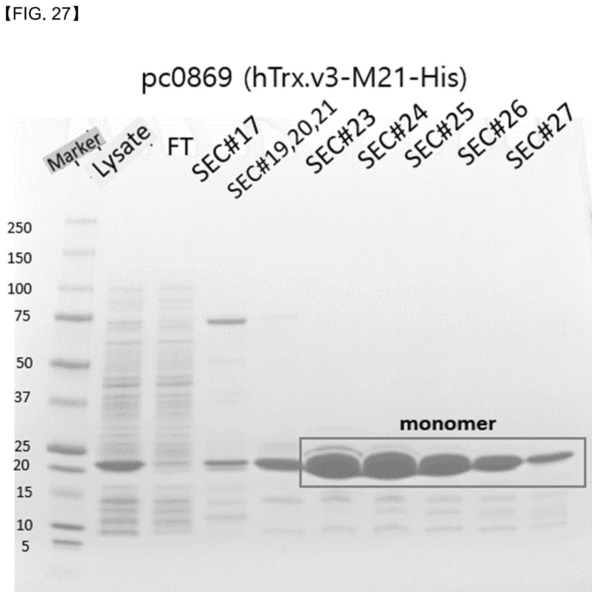

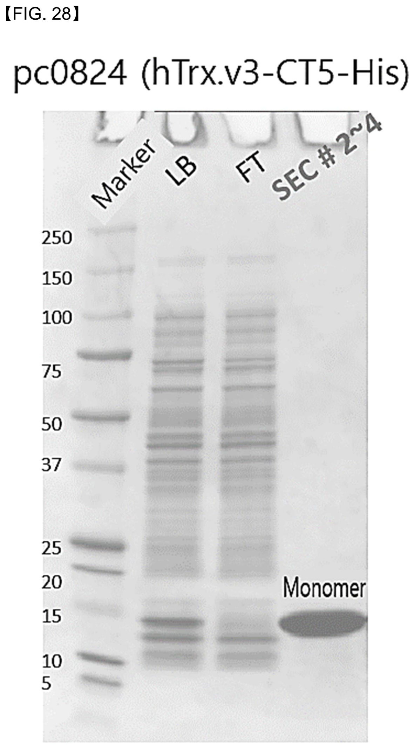

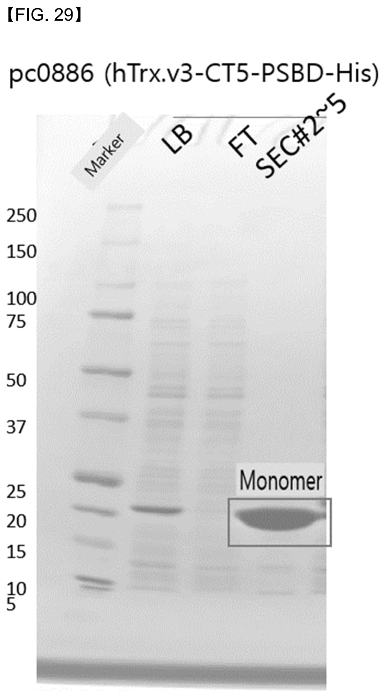

FIG. 24 is the SDS-PAGE results of hTrx.v3-M12-His (pc0811, 5 Cys substitutions), and FIG. 25 is the SDS-PAGE results of hTrx.v3-M12-PSBD-His (pc0883, 5 Cys substitutions, comprising further comprised carrier protein PSBD), and FIG. 26 is the SDS-PAGE results of hTrx.v3-M21-His (pc0812, 5 Cys substitutions), and FIG. 27 is the SDS-PAGE results of hTrx.v3-M21-PSBD-His (pc0869, 5 Cys substitutions, comprising further comprised carrier protein PSBD), and FIG. 28 is the chromatogram results of hTrx.v3-CT5-His (pc0824, 5 Cys substitutions), and FIG. 29 is the chromatogram results of hTrx.v3-CT5-PSBD-His (pc0886, 5 Cys substitutions, comprising further comprised carrier protein PSBD). As shown in FIG. 24 to FIG. 29, it could be confirmed that the monomer band was shown more clearly, and therefore, the purification efficiency of the fusion protein was increased, when the additional carrier protein, PSBD was comprised, regardless of the type of antigen.

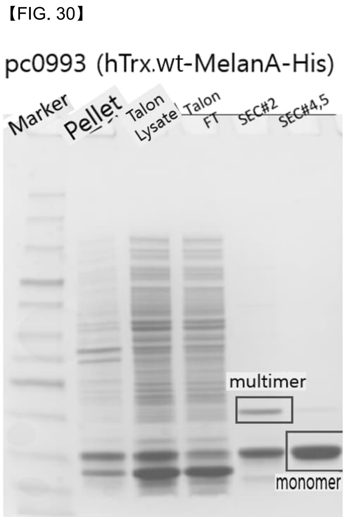

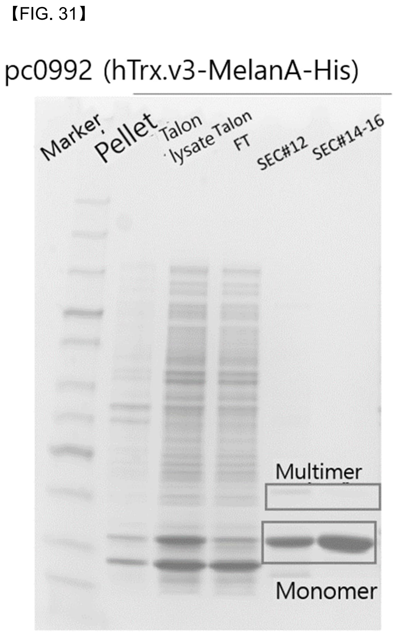

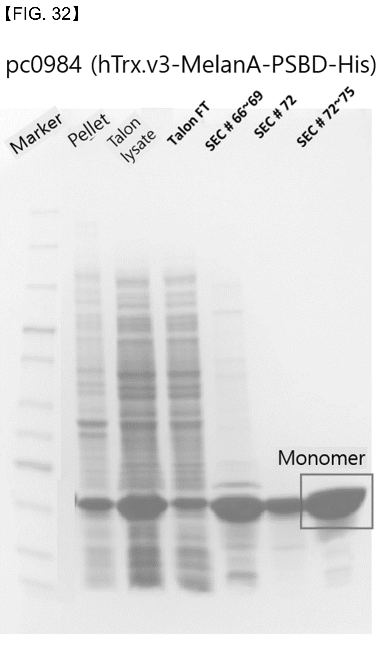

FIG. 30 is the SDS-PAGE results of hTrx.wt-MelanA-His (pc0993, wild type), and FIG. 31 is the SDS-PAGE results of hTrx.v3- MelanA-His (pc0992, 5 Cys substitutions), and FIG. 32 is the SDS-PAGE results of hTrx.v3-MelanA-PSBD-His (pc0984, 5 Cys substitutions, comprising further comprised carrier protein PSBD). As shown in FIG. 30 to FIG. 32, in case of hTrx.wt-MelanA-His (pc0993, wild type), a band corresponding to the multimer was shown, and in case of hTrx.v3- MelanA-His (pc0992, 5 Cys substitutions), the multimer band was faintly shown. On the other hand, in case of hTrx.v3-MelanA-PSBD-His (pc0984, 5 Cys substitutions, comprising further comprised carrier protein PSBD), the multimer band was not shown, and the monomer band was clearly shown. Therefrom, it could be confirmed that when cysteine is substituted, generation of the multimer was reduced during purification, and therefore, the purification efficiency of the fusion protein was improved, and when PSBD was comprised as an additional carrier protein, the purification efficiency of the fusion protein was further improved.

The sterilized final anticancer vaccine active ingredient was stored at -70±10°C.

From the above description, those skilled in the art to which the present disclosure pertains will be able to understand that the present invention can be implemented in other specific forms without changing the technical spirit or essential features thereof. In this regard, the examples described above should be understood as illustrative and non-limiting in all respects. The scope of the present disclosure should be construed that all changed or modified forms derived from the meaning and scope of the claims to be described later and equivalent concepts thereof rather than the above detailed description are included in the scope of the present disclosure.

**Claims**

1.  A fusion protein, comprising

    a peptide antigen, and
    a human thioredoxin protein linked to the N-terminus, C-terminus or both of the peptide antigen,
    wherein at least 3 cysteine residues in the amino acid sequence of the human thioredoxin protein are substituted with non-cysteine residues.

2.  The fusion protein according to claim 1,
    wherein the cysteine residues at the 62nd, 69th and 73rd amino acid positions in the amino acid sequence of the human thioredoxin protein are substituted with non-cysteine residues.

3.  The fusion protein according to claim 1,
    wherein the cysteine residues at the 32nd, 35th, 62nd, 69th and 73rd amino acid positions in the amino acid sequence of the human thioredoxin protein are substituted with non-cysteine residues.

4.  The fusion protein according to claim 1,
    wherein the fusion protein further comprises a carrier protein other than the human thioredoxin at the N-terminus or C-terminus or both.

5.  The fusion protein according to claim 1,

wherein the fusion protein further comprises an affinity tag at the N-terminus, C-terminus, or both.

6. The fusion protein according to claim 1,
   wherein a linker is present between the peptide antigen and the human thioredoxin protein.

7. The fusion protein according to claim 1,
   wherein the peptide antigen comprises a T cell epitope.

8. The fusion protein according to claim 1,
   wherein the peptide antigen comprises a T cell epitope, derived from a tumor antigen, an antigen of an infection source, an autoantigen, or an allergy causing antigen.

9. The fusion protein according to claim 8,
   wherein the tumor antigen comprises a tumor-associated antigen (TAA), a tumor-specific antigen (TSA) or a tumor-derived neoantigen.

10. The fusion protein according to claim 9,
    wherein the tumor-derived neoantigen comprises a mutation specifically expressed in a cancer cell.

11. The fusion protein according to claim 9,
    wherein the tumor-associated antigen (TAA) is CT (Cancer-testis) antigen, EGFR, Melan-A, PSMA(Prostate Specific Membrane Antigen), Survivin, MAGE-A, ADAbp (adenosine deaminase-binding protein), cyclophilin b, gp100, CRC (Colorectal associated antigen)-C017-1 A/GA733, CEA (carcinoembryonic antigen), CAP-1, CAP-2, etv6, AML1, PSA (Prostate Specific Antigen), PSA-1, PSA-2, PSA-3, MAGE (melanoma antigen E), GAGE (G antigen), BAGE (B melanoma antigen), RAGE (renal tumor antigen), LAGE (L antigen), NAG, GnT-V, MUM-1, CDK4, p53, tyrosinase, Muc1 (mucin1), HER2/neu, p21ras, RCAS1, $\alpha$-fetoprotein, E-cadherin, $\alpha$-catenin, $\beta$-catenin, $\gamma$-catenin, p120ctn, PRAME, NY-ESO-1, TRP2, Mammaglobin-A, metallopanstimulin-1 (MPS-1), cytochrome P450 isoform 1B1, 90K/Mac-2 binding protein, Ep-CAM (MK-1), HSP-70, hTERT (TRT), LEA, TAGE-1, 5T4, gp70, SCP-1, c-myc, cyclin B1, MDM2, p62, Koc, IMP1, TA90, OA1, CT-7, HOM-MEL-40/SSX-2, SSX-1, SSX-4, HOM-TES-14/SCP-1, HOM-TES-85, HDAC5, MBD2, TRIP4, NY-CO-45, KNSL6, HIP1R, Seb4D, KIAA1416, IMP1, 90K/Mac-2 binding protein, MDM2, or LMNA.

12. The fusion protein according to claim 8,
    wherein the antigen of the infection source is an antigen derived from a virus, bacterium, parasite, or fungus.

13. The fusion protein according to claim 4,
    wherein the carrier protein is a protein that improves recombinant expression of the peptide antigen, or enhances the purification efficiency of the peptide antigen.

14. The fusion protein according to claim 4,
    wherein the carrier protein further comprised is a protein that improves recombinant expression of the peptide antigen, or enhances the purification efficiency of the peptide antigen.

15. The fusion protein according to claim 4,
    wherein the carrier protein comprised further is one or more selected from NDPK (nucleoside diphosphate kinase B), CSTA (Cystatin-A), Trx (Thioredoxin), RPL7Am (50S ribosomal protein L7Ae), Samp2a (Small archaeal modifier protein 2), TE (Tenascin), TM1112 (*Thermotoga maritima* Cupin_3 domain-containing protein), TrxA (Thioredoxin 1), TTrx (*Thermosipho africanus* Thioredoxin), PSBD (peripheral subunit-binding domain), and fragments thereof.

16. The fusion protein according to claim 5,
    wherein the affinity tag is His or streptavidin.

17. The fusion protein according to claim 6,
    wherein the linker is (GS)n, (G$_2$S)n, (G$_3$S)n, (G$_4$S)n, Gn, LE, SSGG or GGGGSGGGGG (herein, G is Gly, S is Ser, L is Leu, E is Glu, n is an integer of at least 1).

18. The fusion protein according to claim 1,
    wherein the fusion protein has a size of 30kDa or less.

**19.** A nucleic acid molecule encoding the fusion protein of any one claim of claim 1 to claim 18.

**20.** An expression vector comprising the nucleic acid molecule of claim 19.

**21.** A cell transformed with the expression vector of claim 20.

**22.** An immunogenic composition, comprising

the fusion protein of any one claim of claim 1 to claim 18;
a nucleic acid molecule encoding the fusion protein;
an expression vector comprising the nucleic acid molecule; or
a cell transformed with the expression vector.

**23.** The immunogenic composition according to claim 22,
further comprising an adjuvant.

**24.** A composition for enhancing purification efficiency of a peptide antigen, comprising

the fusion protein of any one claim of claim 1 to claim 18;
a nucleic acid molecule encoding the fusion protein;
an expression vector comprising the nucleic acid molecule; or
a cell transformed with the expression vector.

**25.** A composition for preventing or treating cancer, comprising

the fusion protein of any one claim of claim 1 to claim 18;
a nucleic acid molecule encoding the fusion protein;
an expression vector comprising the nucleic acid molecule; or
a cell transformed with the expression vector.

**26.** A thioredoxin variant polypeptide consisting of the amino acid sequence of SEQ ID NO: 7 or SEQ ID NO: 9.

【FIG. 1】

TrxA-M30-His (pc0735)

【FIG. 2】

TrxA.v2-M30-His (pc0802)

【FIG. 3】

hTrx.v2-M30-His (pc0763)

【FIG. 4】

hTrx.v1-M30-His (pc0764)

【FIG. 5】

hTrx.v3-M30-His (pc0792)

【FIG. 6】

hTrx.v3-M30-PSBD-His (pc0890)

【FIG. 7】

【FIG. 8】

hTrx.v1-M44-His (pc0761)

【FIG. 9】

hTrx.v2-M44-His (pc0762)

【FIG. 10】

hTrx.v3-M44-PSBD-His (pc0895)

【FIG. 11】

hTrx.v3-M12-His (pc0811)

【FIG. 12】

hTrx.v3-M12-PSBD-His (pc0883)

【FIG. 13】

hTrx.v3-M21-His (pc0812)

【FIG. 14】

hTrx.v3-M21-PSBD-His (pc0869)

【FIG. 15】

hTrx.v3-CT5-His (pc0824)

Monomer

【FIG. 16】

## hTrx.v3-CT5-PSBD-His (pc0886)

【FIG. 17】

hTrx.wt-MelanA-His (pc0993)

【FIG. 18】

hTrx.v3-MelanA-His (pc0992)

【FIG. 19】

【FIG. 20】

【FIG. 21】

【FIG. 22】

【FIG. 23】

pc0895    (hTrx.v3-M44-PSBD-His)

【FIG. 24】

【FIG. 25】

pc0883 (hTrx.v3-M12-PSBD-His)

【FIG. 26】

pc0812 (hTrx.v3-M21-His)

【FIG. 27】

pc0869 (hTrx.v3-M21-His)

EP 4 644 410 A1

【FIG. 28】

pc0824 (hTrx.v3-CT5-His)

73

【FIG. 29】

pc0886 (hTrx.v3-CT5-PSBD-His)

【FIG. 30】

pc0993 (hTrx.wt-MelanA-His)

【FIG. 31】

pc0992 (hTrx.v3-MelanA-His)

【FIG. 32】

pc0984 (hTrx.v3-MelanA-PSBD-His)

## INTERNATIONAL SEARCH REPORT

| International application No. |
| --- |
| **PCT/KR2023/022048** |

**A. CLASSIFICATION OF SUBJECT MATTER**

**C07K 14/47**(2006.01)i; **C12N 15/62**(2006.01)i; **A61K 39/00**(2006.01)i; **A61P 35/00**(2006.01)i

According to International Patent Classification (IPC) or to both national classification and IPC

**B. FIELDS SEARCHED**

Minimum documentation searched (classification system followed by classification symbols)

C07K 14/47(2006.01); A61K 38/16(2006.01); A61K 39/00(2006.01); C07K 14/00(2006.01); C07K 14/52(2006.01); C07K 14/525(2006.01); C12N 15/62(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 펩티드 항원(peptide antigen), 티오레독신(thioredoxin), 융합 단백질(fusion protein), 시스테인(cysteine), 치환(substitution), C32S, C35S, C62S, C69S, C73S, 정제(purification), 친화성 태그(affinity tag), 신생항원(neoantigen), 캐리어(carrier), 링커(linker)

**C. DOCUMENTS CONSIDERED TO BE RELEVANT**

| Category* | Citation of document, with indication, where appropriate, of the relevant passages | Relevant to claim No. |
| --- | --- | --- |
| Y | KR 10-2018-0129899 A (NEON THERAPEUTICS INC.) 05 December 2018 (2018-12-05) See claims 1-2, 40-41, 70, 77, 83, 95, 187 and 194; and paragraphs [0024], [0048]-[0049], [0156], [0176], [0277], [0288], [0305], [0317], [0327], [0381]-[0382] and [0590]. | 1-25 |
| A | | 26 |
| X | WO 2022-196683 A1 (KM BIOLOGICS CO., LTD. et al.) 22 September 2022 (2022-09-22) See paragraphs [0008], [0023], [0024], [0028], [0037] and [0038]; and SEQ ID NO: 1. | 26 |
| Y | | 1-25 |
| X | NCBI. GenBank accession no. 3KD0_A (12 November 2021). See Reference 2 section and amino acid sequence section. | 26 |
| A | HIROTA, K. et al. AP-1 transcriptional activity is regulated by a direct association between thioredoxin and Ref-1. Proceedings of the National Academy of Sciences. April 1997, vol. 94, pp. 3633-3638. See entire document. | 1-26 |

☑ Further documents are listed in the continuation of Box C.    ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **08 April 2024** | **09 April 2024** |

| Name and mailing address of the ISA/KR | Authorized officer |
| --- | --- |
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
| --- |
| **PCT/KR2023/022048** |

**C. DOCUMENTS CONSIDERED TO BE RELEVANT**

| Category* | Citation of document, with indication, where appropriate, of the relevant passages | Relevant to claim No. |
| --- | --- | --- |
| A | US 9675671 B2 (MCTAVISH, H.) 13 June 2017 (2017-06-13)<br>See entire document. | 1-26 |
| A | CAVAZZINI, D. et al. Enhanced immunogenicity of a positively supercharged archaeon thioredoxin scaffold as a cell-penetrating antigen carrier for peptide vaccines. Frontiers in Immunology. 09 August 2022, vol. 13, thesis no. 958123, pp. 1-15.<br>See entire document. | 1-26 |
| E | WO 2024-025397 A1 (LG CHEM, LTD. et al.) 01 February 2024 (2024-02-01)<br>See claims 1-21; SEQ ID NOS: 47 and 49; and tables 2-3. | 1-26 |

Form PCT/ISA/210 (second sheet) (July 2022)

**INTERNATIONAL SEARCH REPORT**

International application No.

**PCT/KR2023/022048**

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

| International application No. |
| --- |
| **PCT/KR2023/022048** |

| Patent document cited in search report | | | Publication date (day/month/year) | Patent family member(s) | | | Publication date (day/month/year) |
| --- | --- | --- | --- | --- | --- | --- | --- |
| KR | 10-2018-0129899 | A | 05 December 2018 | AU | 2017-240745 | A1 | 04 October 2018 |
| | | | | AU | 2017-240745 | B2 | 19 August 2021 |
| | | | | AU | 2021-269272 | A1 | 09 December 2021 |
| | | | | AU | 2021-269272 | B2 | 06 July 2023 |
| | | | | AU | 2023-241320 | A1 | 26 October 2023 |
| | | | | BR | 112018070183 | A2 | 16 April 2019 |
| | | | | CA | 3018748 | A1 | 05 October 2017 |
| | | | | CN | 109310739 | A | 05 February 2019 |
| | | | | CN | 115558030 | A | 03 January 2023 |
| | | | | EP | 3436048 | A1 | 06 February 2019 |
| | | | | JP | 2019-513373 | A | 30 May 2019 |
| | | | | JP | 2022-116237 | A | 09 August 2022 |
| | | | | KR | 10-2022-0163523 | A | 09 December 2022 |
| | | | | US | 2019-0307868 | A1 | 10 October 2019 |
| | | | | US | 2023-0405102 | A1 | 21 December 2023 |
| | | | | US | 2023-0414735 | A1 | 28 December 2023 |
| | | | | WO | 2017-173321 | A1 | 05 October 2017 |
| WO | 2022-196683 | A1 | 22 September 2022 | CN | 117321208 | A | 29 December 2023 |
| | | | | EP | 4310187 | A1 | 24 January 2024 |
| US | 9675671 | B2 | 13 June 2017 | AU | 2015-204540 | A1 | 04 August 2016 |
| | | | | AU | 2015-204540 | B2 | 19 March 2020 |
| | | | | CA | 2936675 | A1 | 16 July 2015 |
| | | | | CA | 2936675 | C | 27 June 2023 |
| | | | | EP | 3094339 | A2 | 23 November 2016 |
| | | | | EP | 3094339 | B1 | 11 December 2019 |
| | | | | JP | 2017-503862 | A | 02 February 2017 |
| | | | | JP | 6835590 | B2 | 24 February 2021 |
| | | | | US | 10391147 | B2 | 27 August 2019 |
| | | | | US | 10967049 | B2 | 06 April 2021 |
| | | | | US | 2015-0196656 | A1 | 16 July 2015 |
| | | | | US | 2017-0266260 | A1 | 21 September 2017 |
| | | | | US | 2018-0021408 | A1 | 25 January 2018 |
| | | | | US | 2019-0336580 | A1 | 07 November 2019 |
| | | | | US | 9801923 | B2 | 31 October 2017 |
| | | | | WO | 2015-106224 | A2 | 16 July 2015 |
| | | | | WO | 2015-106224 | A3 | 12 November 2015 |
| WO | 2024-025397 | A1 | 01 February 2024 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9014837 A
- WO 9313302 A

- WO 9219265 A

**Non-patent literature cited in the description**

- **KARLIN** ; **ALTSCHUL**. *Pro. Natl. Acad. Sci. USA*, 1993, vol. 90, 5873

- **PEARSON**. *Methods Enzymol.*, 1990, vol. 183, 63